(19) Europäisches Patentamt
European Patent Office
Office européen des brevets

(11) Publication number: **0 126 970**
**A2**

(12) **EUROPEAN PATENT APPLICATION**

(21) Application number: 84104587.5

(22) Date of filing: 24.04.84

(51) Int. Cl.³: C 07 D 495/04
C 07 D 495/14, A 61 K 31/435
//C07D333/36, (C07D495/04, 333/00, 221/00), (C07D495/14, 333/00, 231/00, 221/00)

(30) Priority: 27.04.83 GB 8311426
20.07.83 GB 8319539
03.11.83 GB 8329370
17.11.83 GB 8330669
29.11.83 GB 8331827

(43) Date of publication of application: 05.12.84 Bulletin 84/49

(84) Designated Contracting States: BE CH DE FR GB IT LI NL SE

(71) Applicant: BEECHAM GROUP PLC
Beecham House Great West Road
Brentford Middlesex TW8 9BD(GB)

(72) Inventor: Thompson, Mervyn
7 Hawkenbury
Harlow Essex, CM19 4HZ(GB)

(72) Inventor: Forbes, Ian Thomson
16 Sutcliffe Close
Stevenage Hertfordshire, SG1 5PJ(GB)

(74) Representative: Stott, Michael John et al,
European Patent Attorney Beecham Pharmaceuticals
Great Burgh Yew Tree Bottom Road
Epsom Surrey KT18 5XQ(GB)

(54) **Anxiolytic and anti-depressant thienopyridine derivatives.**

(57) Compounds of formula (I) and pharmaceutically acceptable salts thereof:

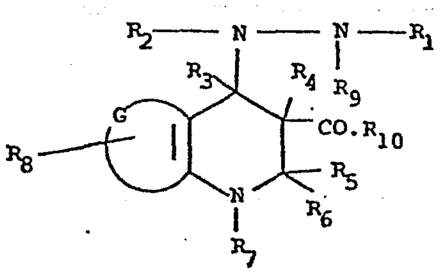

(I)

wherein:

G together with the two carbon atoms to which it is bonded is a thieno moiety;

$R_1$ is phenyl optionally substituted by one or more $C_{1-6}$ alkyl, $C_{1-6}$ alkoxy, $C_{1-6}$ alkylthio, hydroxy, $C_{2-7}$ alkanoyl, halo, trifuluoromethyl, nitro, amino optionally substituted by one or two $C_{1-6}$ alkyl groups or by $C_{2-7}$ alkanoyl, cyano, carbamoyl or carboxy groups; or pyridyl optionally substituted by $C_{1-6}$ alkyl or halo;

$R_6$ is hydrogen, $c_{1-6}$ alkyl or phenyl optionally substituted as defined hereinbefore for $R_1$ when phenyl;

$R_8$ is hydrogen, one of the optional substituents recited hereinbefore for $R_1$ when phenyl or phenyl optionally substituted as defined hereinbefore for $R_1$ when phenyl; and either $R_2$ is hydrogen, or $C_{1-6}$ alkyl optionally substituted by hydroxy, amino disubstituted by $C_{1-6}$ alkyl, or phenyl optionally substituted as defined hereinbefore for $R_1$ when phenyl;

$R_3$ and $R_4$ together represent a bond;

$R_5$ and $R_7$ together represent a bond; and

$R_9$ is hydrogen and $R_{10}$ is hydroxy, $C_{1-6}$ alkoxy or amino optionally substituted by one or two independently selected $C_{1-6}$ alkyl groups or by phenyl optionally substituted as defined hereinbefore for $R_1$ when phenyl, or $R_9$ and $R_{10}$ together represent a bond;

or

$R_2$ and $R_3$ together represent a bond;

$R_4$ and $R_5$ together represent a bond; and

$R_7$ is hydrogen, $C_{1-6}$ alkyl optionally substituted by hydroxy, amino disubstituted by $c_{1-6}$ alkyl, or phenyl optionally substituted as defined hereinbefore for $R_1$ when phenyl;

and

$R_9$ and $R_{10}$ together represent a bond, having pharmacological activity, a process and intermediates for their preparation, compositions containing them and their use in the treatment of mammals.

EP 0 126 970 A2

TITLE MODIFIED
see front page



0126970



# NOVEL COMPOUNDS

This invention relates to novel compounds having pharmacological activity, to a process for their preparation, to compositions containing them and to their use in the treatment of mammals.

U.S. Patent 4,312,870 discloses 2-aryl-pyrazolo [4,3-c]quinolin-3-(1 and 5H)-ones of formulae (A) and (B):

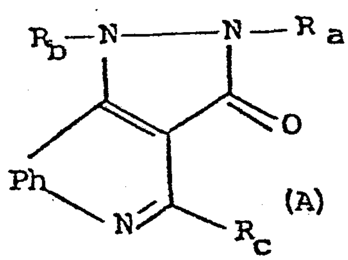
(A)

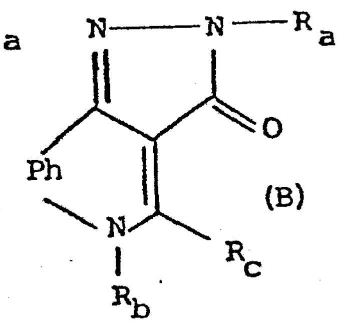
(B)

wherein Ph is 1,2-phenylene, unsubstituted or substituted by up to 3 identical or different members selected from lower alkyl, lower alkoxy, lower alkylthio, hydroxy, halogeno, trifluoromethyl, nitro, amino, mono or di-lower alkylamino, cyano, carbamoyl and carboxy. $R_a$ is unsubstituted or substituted phenyl as defined by H-Ph, pyridyl, lower alkylpyridyl, or halogenopyridyl; $R_b$ is hydrogen, lower alkyl or lower (hydroxy, dialkylamino or H-Ph)alkyl; and $R_c$ is

hydrogen or lower alkyl; their 3-hydroxy-tautomers, lower alkanoyl, carbamoyl, mono- or di-lower alkylcarbamoyl derivatives of the said (hydroxy or amino)-(phenyl or phenylene)compounds, or pharmaceutically acceptable salts thereof; useful in the treatment of anxiety or depression in mammals.

A class of thienopyridines has now been discovered which compounds have CNS activity, in particular anxiolytic and/or anti-depressant activity.

Accordingly, the present invention provides compounds of formula (I) and pharmaceutically acceptable salts thereof:

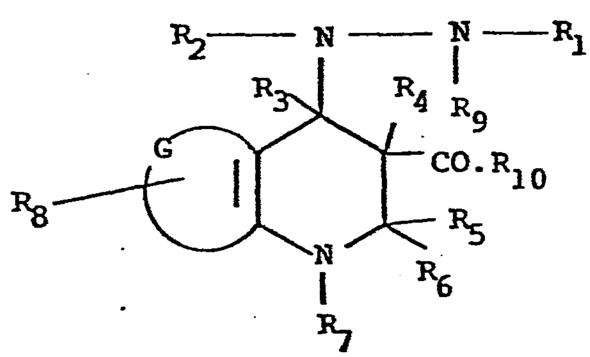

(I)

wherein:

G together with the two carbon atoms to which it is bonded is a thieno moiety;

$R_1$ is phenyl optionally substituted by one or more

$C_{1-6}$ alkyl, $C_{1-6}$ alkoxy, $C_{1-6}$ alkylthio, hydroxy, $C_{2-7}$ alkanoyl, halo, trifluoromethyl, nitro, amino optionally substituted by one or two $C_{1-6}$ alkyl groups or by $C_{2-7}$ alkanoyl, cyano, carbamoyl or carboxy groups; or pyridyl optionally substituted by $C_{1-6}$ alkyl or halo;

$R_6$ is hydrogen, $C_{1-6}$ alkyl or phenyl optionally substituted as defined hereinbefore for $R_1$ when phenyl;

$R_8$ is hydrogen, one of the optional substituents recited hereinbefore for $R_1$ when phenyl or phenyl optionally substituted as defined hereinbefore for $R_1$ when phenyl; and either

$R_2$ is hydrogen, or $C_{1-6}$ alkyl optionally substituted by hydroxy, amino disubstituted by $C_{1-6}$ alkyl, or phenyl optionally substituted as defined hereinbefore for $R_1$ when phenyl;

$R_3$ and $R_4$ together represent a bond;

$R_5$ and $R_7$ together represent a bond; and

$R_9$ is hydrogen and $R_{10}$ is hydroxy, $C_{1-6}$ alkoxy or amino optionally substituted by one or two independently selected $C_{1-6}$ alkyl groups or by phenyl optionally substituted as defined hereinbefore for $R_1$ when phenyl, or $R_9$ and $R_{10}$ together represent a bond;

or

$R_2$ and $R_3$ together represent a bond;

$R_4$ and $R_5$ together represent a bond; and

$R_7$ is hydrogen, $C_{1-6}$ alkyl optionally substituted by hydroxy, amino disubstituted by $C_{1-6}$ alkyl, or phenyl optionally substituted as defined hereinbefore for $R_1$ when phenyl;

and

$R_9$ and $R_{10}$ together represent a bond.

Values for $R_1$ include unsubstituted phenyl and phenyl substituted by one or two methyl, ethyl, _n_- or _iso_-propyl, methoxy, ethoxy, _n_- or _iso_-propoxy, methylthio, ethylthio, _n_- or _iso_-propylthio, hydroxy, acetyl, propionyl, fluoro, chloro, bromo, trifluoromethyl, nitro or cyano or 2-pyridyl optionally substituted by chloro, bromo, methyl, ethyl, _n_ or _iso_-propyl.

Often $R_1$ is unsubstituted phenyl, phenyl substituted by halogen, nitro, $C_{1-6}$ alkyl or $C_{1-6}$ alkoxy, or unsubstituted pyridyl.

Values for $R_6$ include hydrogen, methyl, ethyl, _n_- and _iso_-propyl, and phenyl. Preferably $R_6$ is hydrogen, methyl or phenyl.

Values for $R_8$ include hydrogen and the optional phenyl substituents described for $R_1$ above. Often $R_8$ is hydrogen, methyl, ethyl or chloro.

Values for $R_2$ when other than, together with $R_3$, a bond, include hydrogen, methyl, ethyl, and _n_- and _iso_-propyl, and methyl, ethyl and _n_- and _iso_-propyl substituted by hydroxy, di($C_{1-3}$) alkylamino or phenyl, wherein the hydroxy or amino group is separated from

the ring nitrogen atom by at least two carbon atoms, such as 2-(hydroxy, dimethylamino or diethylamino)-ethyl, 2-or 3-(hydroxy or dimethylamino)-propyl, benzyl and 1-or 2-phenethyl.

Values for $R_7$ when other than together with $R_5$, being a bond are as described above for $R_2$.

Particular values for $R_7$ are hydrogen, methyl and dimethylaminopropyl.

When $R_9$ and $R_{10}$ together represent a bond, then preferably $R_2$ and $R_3$ represent a bond and $R_4$ and $R_5$ represent a bond. $R_7$ is then preferably hydrogen.

When $R_9$ is hydrogen and $R_{10}$ is hydroxy, $C_{1-6}$ alkoxy or amino optionally substituted as hereinbefore defined, values of $R_{10}$ include hydroxy, methoxy, ethoxy and _n_- and _iso_-propoxy. Often $R_{10}$ is methoxy or ethoxy, in particular ethoxy.

It will be appreciated that when $R_2$ is hydrogen or when $R_9$ and $R_{10}$ together represent a bond and $R_7$ is hydrogen, the compounds of formula (I) may exist tautomerically in more than one form. The invention extends to each of these forms and to mixtures thereof.

The compounds of formula (I) may form pharmaceutically acceptable acid addition salts with conventional acids such as hydrochloric, hydrobromic, phosphoric, sulphuric, citric, tartaric or lactic acid. The compounds of formula (I) wherein $R_7$ is hydrogen may also form salts with strong bases e.g. with alkali metals such as sodium or potassium, although these are not in general pharmaceutically acceptable. The compounds of formula (I) wherein $R_9$ is

hydrogen and $R_{10}$ is hydroxy may also form pharmaceutically acceptable salts with bases e.g. with alkali metals such as sodium or potassium, with alkaline earth metals, and optionally substituted ammonium salts.

There is a group of compounds within formula (I) wherein the thieno moiety formed by G and the two carbon atoms to which it is bonded is fused along its 2,3-face to the pyridine or dihydropyridine ring depicted in formula (I), in either the [2,3-b] or [3,2-b] orientation.

In a sub-group of compounds within this group the thieno moiety is fused in the [3,2-b] orientation.

In a class of compounds within this sub-group $R_9$ and $R_{10}$ together represent a bond, $R_6$ is hydrogen or $C_{1-6}$ alkyl and $R_8$ is hydrogen or one of the optional sustituents recited hereinbefore for $R_1$ when phenyl.

In a second class of compounds within this sub-group $R_9$ is hydrogen and $R_{10}$ is hydroxy or $C_{1-6}$ alkoxy, R2 is hydrogen, $R_6$ is hydrogen or $C_{1-6}$ alkyl, and $R_8$ is hydrogen or one of the optional substituents recited hereinbefore for $R_1$ when phenyl.

There is another group of compounds within formula (I) wherein the aforementioned thieno moiety is fused along its 3,4 face to the b face of the aforementioned pyridine or dihydropyridine ring.

There is a group of compounds within formula (I) of formula (II):

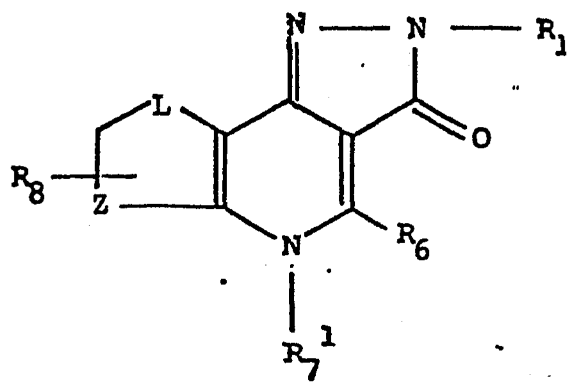

(II)

wherein

one of L and Z is a sulphur atom and the other is a carbon atom doubly bonded to the carbon atom between L and Z;

$R_7^1$ is hydrogen, $C_{1-6}$ alkyl optionally substituted by hydroxy, amino disubstituted by $C_{1-6}$ alkyl, or phenyl optionally substituted as defined hereinbefore for $R_1$;
and the remaining variable groups are as defined in formula (I).

Examples of and preferred values for R1, R6, $R_7^1$ and $R_8$ are as described for the corresponding variables under formula (I).

There is a sub-group of compounds within formula (II) of formula (III):

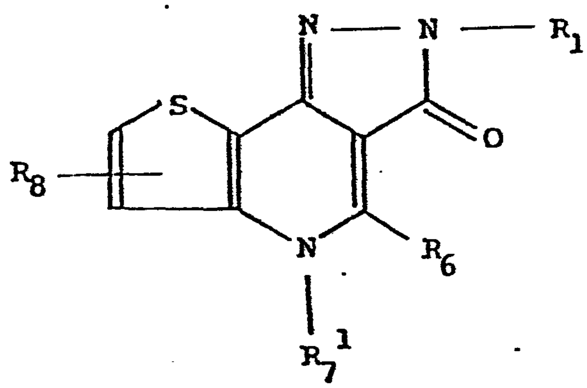

(III)

wherein the variable groups are as defined in formula (II).

Examples of, and preferred, values for $R_1$, $R_6$, $R_7^1$ and $R_8$ are as described under formula (II). Often $R_8$ is hydrogen.

There is a preferred class of compounds within formula (III) of formula (IV):

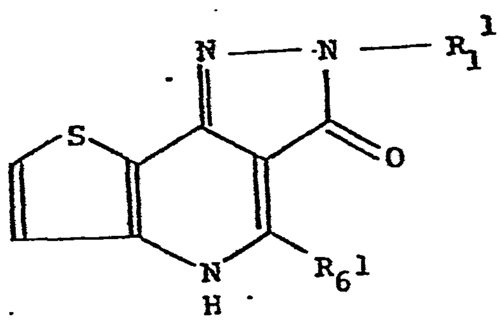

(IV)

wherein

$R_1^1$ is phenyl optionally substituted by $C_{1-6}$ alkyl, $C_{1-6}$ alkoxy, hydroxy, fluoro, chloro, bromo, trifluoromethyl, nitro, cyano or amino optionally substituted as defined in formula (I) for $R_1$; and

$R_6{}^1$ is hydrogen, methyl or phenyl.

$R_1{}^1$ may be phenyl optionally substituted by halo, $C_{1-6}$ alkyl, or $C_{1-6}$ alkoxy,or pyridyl.

$R_1{}^1$ is favourably phenyl substituted by fluoro, chloro or bromo, often phenyl <u>para</u>-substituted by chloro.

$R_6{}^1$ may be hydrogen, methyl or phenyl.

There is a further sub-group of compounds within formula (II) of formula (V):

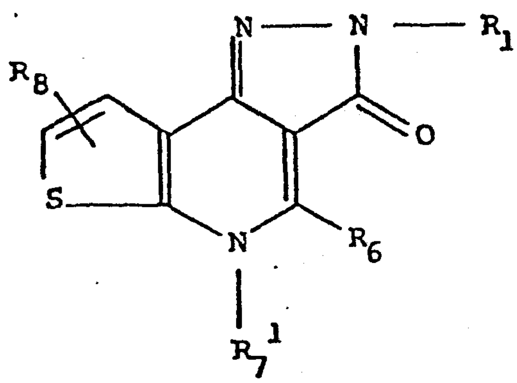

(V)

wherein the variable groups are as defined in formula (II).

Examples of, and preferred values for R1, R6, $R_7{}^1$ and $R_8$ are as described under formula (II) R8 is often methyl or ethyl. $R_6$ is often methyl or hydrogen.

There is a preferred class of compounds within formula (V) of formula (VI):

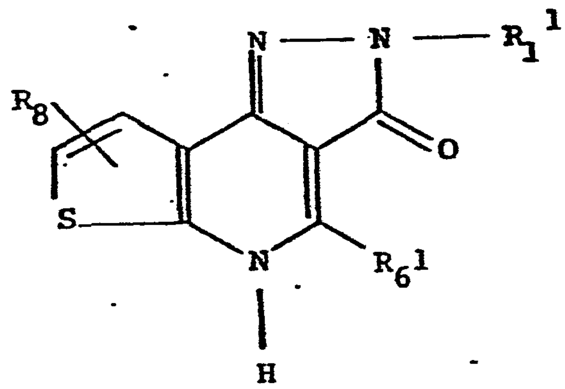

(VI)

wherein the variable groups are as hereinbefore defined.

$R_1^1$ is favourably phenyl substituted by fluoro, chloro or bromo, often phenyl para-substituted by chloro.

$R_6^1$ is favourably hydrogen or methyl.

Examples of values for $R_8$ are as described under formula (V).

Another group of compounds within formula (I) is of formula (VII):

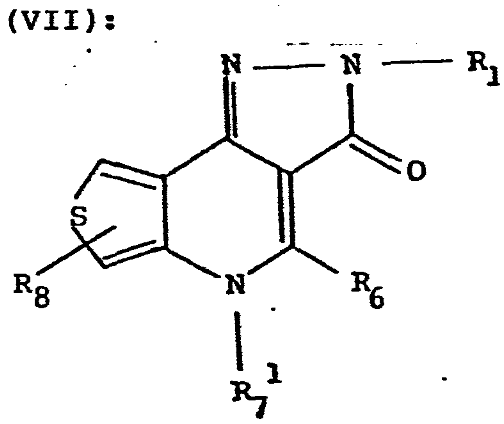

(VII)

wherein the variable groups are as defined in formula (II).

Examples of, and preferred, values for $R_1$, $R_6$, $R_7^1$ and $R_8$ are as described for the corresponding variables under formula (I).

There is a class of compounds within formula (VII) of formula (VIII):

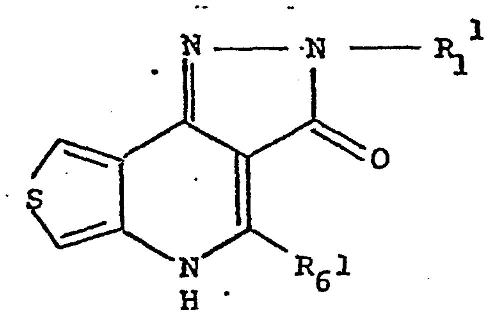

(VIII)

wherein the variable groups are as hereinbefore defined.

Favourable values for $R_1^1$ are as described under formula (IV).

$R_6^1$ may be hydrogen.

There is a further group of compounds within formula (I) of formula (IX):

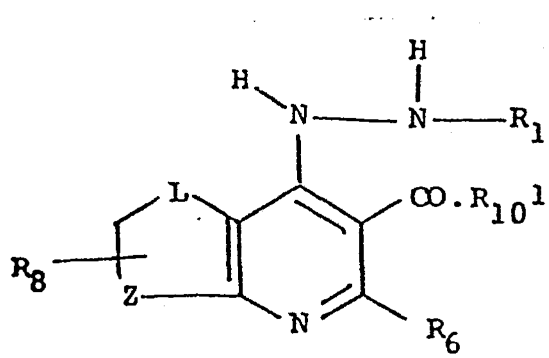

(IX)

wherein $R_{10}^{1}$ is hydroxy, $C_{1-6}$ alkoxy or amino optionally substituted as hereinbefore defined; and the remaining variable groups are as defined in formula (II).

Examples of, and preferred values for $R_1$, $R_6$, $R_8$ and $R_{10}^{1}$ are as described for the corresponding variables under formula (I).

There is a sub-group of compounds within formula (IX) of formula (X):

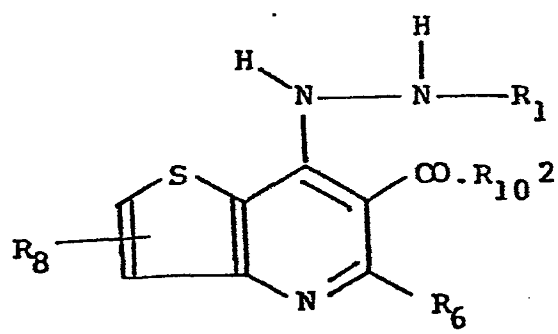

(X)

wherein $R_{10}^{2}$ is hydroxy or $C_{1-6}$ alkoxy and the remaining variable groups are as defined in formula (IX).

Examples of, and preferred, values for $R_1$, $R_6$, $R_8$ are as described under formula (IX). Often $R_8$ is hydrogen, methyl or chloro. $R_{10}^{2}$ may be methoxy or ethoxy.

There is a preferred class of compounds within formula (X) of formula (XI):

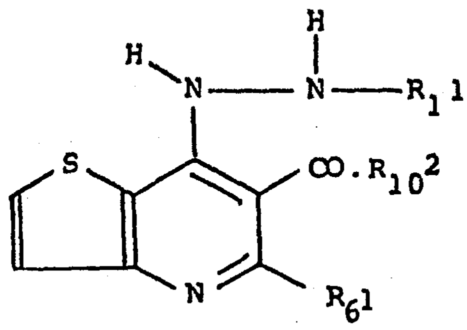

(XI)

wherein
$R_1{}^1$ and $R_6{}^1$ are as defined in formula (IV), and $R_{10}{}^2$ is as defined in formula (X).

$R_1{}^1$ may be phenyl optionally substituted by halo, $C_{1-6}$ alkyl, $C_{1-6}$ alkoxy or nitro, or pyridyl.

$R_1{}^1$ is favourably unsubstituted phenyl or phenyl substituted by fluoro, chloro, or bromo, or $C_{1-6}$ alkyl in particular phenyl para-substituted by iso-propyl.

$R_6{}^1$ may be hydrogen, methyl or phenyl.

There is a further sub-group of compounds within formula (IX) of formula (XII):

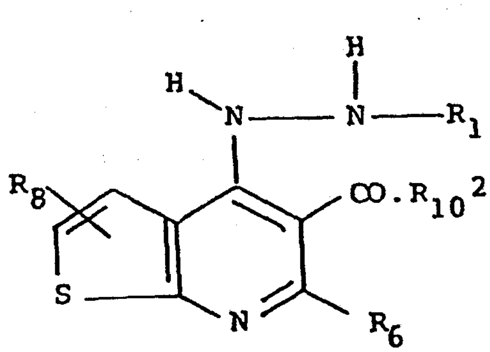

(XII)

wherein the variable groups are as defined in formula (X).

Examples of, and preferred values for $R_1$, $R_6$, $R_8$ and $R_{10}^2$ are as described under formula (X). $R_8$ is often ethyl. $R_6$ is often hydrogen.

There is a preferred class of compounds within formula (XII) of formula (XIII):

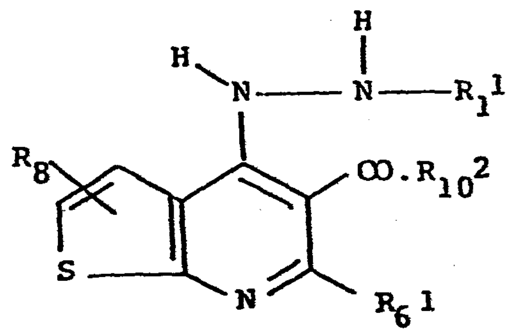

(XIII)

wherein the variable groups are as hereinbefore defined.

$R_1^1$ is favourably unsubstituted phenyl or phenyl substituted by fluoro, chloro, bromo or $C_{1-6}$ alkyl, often phenyl <u>para</u>-substituted by chloro.

$R_6^1$ is favourably hydrogen or methyl.

Examples of values for $R_{10}^2$ are as described under formula (X). Examples of $R_8$ include ethyl.

Yet another group of compounds within formula (I) is of formula (XIV):

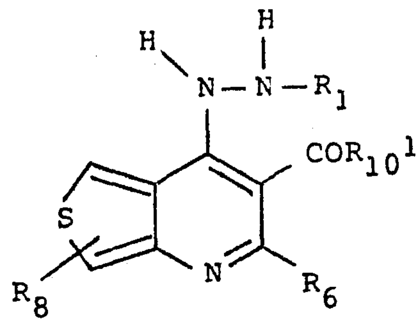

(XIV)

wherein the variable groups are as defined in formula (IX).

Examples of, and preferred, values for $R_1$, $R_6$, $R_8$ and $R_{10}^1$ are as described for corresponding variables under formula (I).

There is a class of compounds within formula (XIV) of formula (XV):

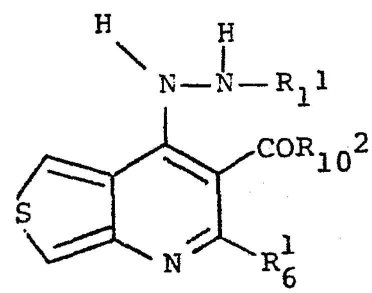

(XV)

wherein the variable groups are as hereinbefore defined.

Favourable values of $R_1^1$ and $R_{10}^2$ are as described under formula (XI).

$R_6^1$ may be hydrogen.

The invention also provides a process for the preparation of a compound of formula (I) or a pharmaceutically acceptable salt thereof which process comprises the reaction of a compound of formula (XVI):

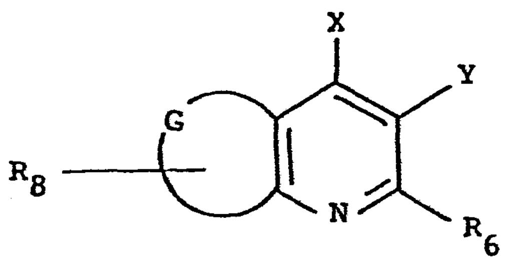

(XVI)

or a salt thereof,

wherein

i) when $R_9$ and $R_{10}$ in the desired compound of formula (I) are hydrogen and $R_{10}{}^1$ as hereinbefore defined respectively,

X is halo;

Y is $COR_{10}{}^1$ as hereinbefore defined or nitrile; and the remaining variables are as hereinbefore defined;

with a compound of formula $R_2HN\text{-}NH\text{-}R_1$ where $R_1$ and $R_2$ are as hereinbefore defined;

and thereafter, in the resultant compound, when Y is nitrile, converting Y to $COR_{10}{}^1$ as hereinbefore defined; optionally converting $R_{10}{}^1$ to other $R_{10}{}^1$;

ii) when $R_9$ and $R_{10}$ in the desired compound of formula (I) together represent a bond;

a) X is $NR_2$-NH-$R_1$ where $R_1$ and $R_2$ are as hereinbefore defined and Y is $COR_{11}$ where $R_{11}$ is halo or Y is $COR_{10}{}^2$ where $R_{10}{}^2$ is as hereinbefore defined and the compound of formula (XVI) or the salt thereof optionally prepared by process variant i) hereinbefore optionally followed by salification;

b) X is halo, and Y is $CON(NR_2R_{15})R_1$ where $R_1$ and $R_2$ are as hereinbefore defined; and $R_{15}$ is hydrogen or a labile deactivating N-protecting group;

c) X is $C_{1-6}$ alkoxyamino or azido and Y is $CONHR_1$ where $R_1$ is as hereinbefore defined;

to cyclise;

and thereafter, in the resultant compound of formula (I) wherein $R_9$ and $R_{10}$ together represent a bond; optionally converting $R_2$ or $R_7$ hydrogen to other $R_2$ or $R_7$;

and, in the resultant compound of formula (I), optionally converting $R_8$ to other $R_8$; and optionally forming a pharmaceutically acceptable salt.

Suitable salts of those compounds of formula (XVI) which can form salts include the salts listed hereinbefore as examples of pharmaceutically acceptable salts for compounds of formula (I).

Suitable values for X in process variant i) (when it is halo) include chloro and bromo, preferably chloro.

The reaction of process variant i) of a compound of formula (XVI) or a salt thereof with $R_2HN-NH-R_1$ where $R_1$ and $R_2$ are as hereinbefore defined is generally carried out with the compound itself rather than with any salt it may form. In this case the reaction may be carried out in an inert solvent, such as a lower alkanol for example ethanol, or in a mixture of such solvents. The reaction may conveniently be effected at a slightly elevated temperature, for example in the range 60° to 100°C, such as between 70° and 90°C, and most conveniently at the boiling point of the reaction mixture. However, it will be appreciated that when Y in the compound of formula (XVI) or the salt thereof is $COR_{10}{}^2$ as hereinbefore defined, that is, carboxyl or $C_{1-6}$ alkoxycarbonyl, the resultant compound of formula (I) or the salt thereof may serve as a starting material for process variant ii) a). In general the temperature for the cyclisation of the resultant compound from process variant i) will be higher than a convenient reaction temperature for its formation in process variant i) and the isolation of the desired product of process variant i) presents no problems.

However, in order to isolate some resultant compounds of formula (I) wherein $R_9$ is hydrogen and $R_{10}$ is hydroxy or $C_{1-6}$ alkoxy before they cyclise to corresponding compounds of formula (I) wherein $R_9$ and $R_{10}$ together are a bond, the skilled man will appreciate that it may be necessary to effect reaction at a substantially lower temperature.

When Y is nitrile in the compound of formula (XVI) or its salts, the corresponding group in the compound resulting from the foregoing reaction must be

subsequently converted to $COR_{10}{}^{1}$ as hereinbefore defined.

When $R_{10}{}^{1}$ is amino or hydroxyl, conversion may be achieved by conventional hydrolysis of the nitrile group, adjusting the reaction conditions conventionally to obtain the desired product.

An $R_{10}{}^{1}$ hydroxyl group may be subsequently converted to an $R_{10}{}^{1}$ $C_{1-6}$ alkoxy group by conventional esterification.

As regards the optional subsequent interconversion of $R_{10}{}^{1}$ substituents in the compound of formula (I) resulting from process variant i), some of these are discussed immediately hereinbefore. Other such interconversions include the conversion of $R_{10}{}^{1}$ $C_{1-6}$ alkoxy or amino to hydroxyl by conventional deesterification and amide hydrolysis respectively.

All such interconversions may of course also be effected for corresponding groups in all intermediates in the synthetic route to the compounds of formula (I) and their salts, in particular in intermediates of formulae (XVI) to (XVIII).

In process variant ii) a), as mentioned hereinbefore, when X is $R_2N\text{-}NH\text{-}R_1$ where $R_1$ and $R_2$ are as hereinbefore defined and Y is $COR_{10}{}^{2}$ where $R_{10}{}^{2}$ is as hereinbefore defined, the compounds of formula (XVI) is also of formula (I). The cyclisation of the compound or a salt thereof to a compound of formula (I) wherein $R_9$ and $R_{10}$ together are a bond, or a salt thereof, may be effected by heating the compound or its salt to a temperature in the range of 70° to 180°C, advantageously in an inert liquid such as a lower alkanol for example ethanol or sec-butanol, or an

aliphatic or aromatic hydrocarbon or aromatic ether, such as toluene, xylene, biphenyl or diphenyl ether, or a mixture of such liquids. The liquid or liquid mixture is preferably a solvent for the compound of formula (XVI) or the salt thereof, where a liquid or liquid mixture is used. The reaction may most conveniently be effected at the boiling point of the reaction mixture. It is advantageous to use an inert solvent with a boiling point above that of the water or alkanol generated in the cyclisation reaction and to distil off that water or alkanol during the reaction.

It is possible to generate the intermediate compound of formula (XVI) wherein X is $R_2N\text{-}NH\text{-}R_1$ where $R_1$ and $R_2$ are as hereinbefore defined and $R_{10}$ is hydroxy or $C_{1-6}$ alkoxy or the salt thereof and to cyclise it in situ without isolation in a one-pot process. In this case it is often convenient to effect the first step in a relatively low boiling inert solvent, such as a lower alkanol, for example ethanol, at the boiling point of the reaction mixture, and then to add a higher boiling inert solvent such as an aliphatic or aromatic hydrocarbon or an aromatic ether and to effect the second step at the boiling point of that reaction mixture. Where the water or lower alkanol generated in the second step is distilled off,any solvent lower alkanol will of course also distil off.

Alternatively it is convenient to produce the compound of formula (XVI) or in particular the salt therof by process variant i) to isolate the compound or its salt for use in process variant ii) a). In this case it is convenient to effect the cyclisation in a relatively low boiling inert solevent, such as a lower alkanol for example ethanol or sec-butanol, at the boiling point of the reaction mixture under reflux.

The reaction may advantageously be effected in the presence of a base, such as a mild inorganic base, for example potassium carbonate. When an acid addition salt of a compound of formula (XVI) is used, it is advantageous to use more than one equivalent of base.

As an alternative to the direct cyclisation of a compound of formula (XVI) or a salt thereof wherein X is $R_2N$-NH-$R_1$ and Y is $COR_{10}{}^2$ where $R_1$ and $R_{10}{}^2$ are as hereinbefore defined, such a compound wherein Y is carboxy or a salt of the compound may be halogenated conventionally to form the corresponding acid halide, and this acid halide cyclised under conditions similar to those described hereinbefore for process variant i). The acid halide or its salt may cyclise in situ on formation in the halogenation process.

In process variant ii) b), examples of $R_{15}$ when a labile deactivating N-protecting group include trifluoroacetyl.

In process variant ii) b), the acid hydrazides of formula (XVI) or their salts may be cyclised in solvents and at temperatures similar to those for process variant ii) a). Advantageously the reaction is effected under basic conditions, in order to neutralize the generated hydrohalic acids, for example in the presence of an alkali metal hydroxide and water.

In process variant ii) c) the ring-closure of the amides of formula (XVI) or their salts occurs by heating them to a temperature between 120° and 300°, preferably between 200° and 250°, advantageously in one or more inert solvents.

The optional subsequent conversion of $R_2$ or $R_7$ when hydrogen in the resultant cyclised compound of formula (I) wherein $R_9$ and $R_{10}$ together are a bond to other $R_2$ or $R_7$ may be effected conventionally using respectively a compound of formula $(R_2{}^1)_2Q_1$or $R_7{}^1Q_2$ wherein $R_2{}^1$ and $R_7{}^1$ are each $C_{1-6}$ alkyl optionally substituted by hydroxy or amino disubstituted by $C_{1-6}$ alkyl, or by phenyl optionally substituted as defined hereinbefore for $R_1$, and $Q_1$ is a reactive divalent ester group, and $Q_2$ is halo or a reactive monovalent ester group. Examples of $Q_1$ include sulphate. Examples of $Q_2$ include mesyloxy and tosyloxy, and in particular halo such as iodo.

Groups $R_8$ may be interconverted in all the resultant compounds of formula (I) by methods generally known in the art of aromatic chemistry, although such interconversion is desirably avoided.

Pharmaceutically acceptable acid addition salts of the resultant compound of formula (I) may be formed conventionally, for example by treatment of the compound with the corresponding acid. Pharmaceutically acceptable salts at the $COR_{10}$ carboxyl group of some compounds of formula (I) may also be found conventionally, for example by treatment of the compound with a corresponding base.

An intermediate of the formula (XVI) in process variant i) that is wherein X is halo and Y is CO $R_{10}{}^{1}$ as hereinbefore defined or nitrile may be prepared by reaction of a compound of formula (XVII):

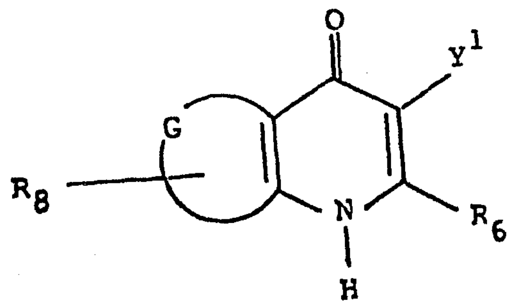

(XVII)

wherein $Y^1$ is $COR_{10}{}^{1}$ as hereinbefore defined or nitrile, and the variables are as hereinbefore defined, with a halogenating agent.

Suitable halogenating agents include phosphorus oxychloride, phosphorus oxybromide, thionyl chloride and phosphorus pentachloride. Phosphorus oxychloride is a preferred halogenating agent.

It will be appreciated that, if a compound of formula (XVI) wherein $Y^1$ is carboxy or amino optionally substituted as herein before defined is desired, then $Y^1$ is preferably not corresponding carboxy or amino optionally substituted as hereinbefore defined in the compound of formula (XVII) because of possible halogenation of $Y^1$.

Thus, where the desired compound of formula (XVI) contains a $Y^1$ carboxy group, $Y^1$ in the compound of formula (XVII) is preferably $C_{1-6}$ alkoxycarbonyl or nitrile, which may be hydrolysed to carboxyl in the resultant compound of formula (XVI) as described hereinbefore.

However when $Y^1$ in the desired compound of formula (XVI) is amino optionally substituted as hereinbefore defined, use may be made of the halogenation of $Y^1$ when carboxy in the compound of formula (XVII), to give a compound of formula (XXV) (see hereinafter). The compound of formula (XXV) then may be reacted conventionally with ammonia optionaly substituted as hereinbefore defined for $Y^1$ when amino to give the desired compound of formula (XVII).

Compounds of the formula (XVII) where G is L.C.Z.as in formula (II) where L and Z are as hereinbefore defined may be prepared in accordance with known procedures, for example by the cyclisation of a compound of formula (XVIII):

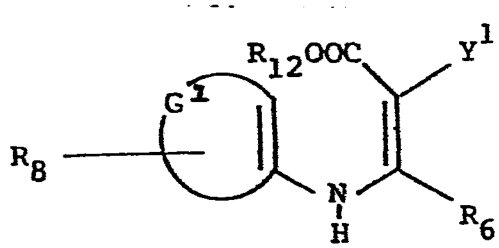

(XVIII)

wherein $G_1$ is L.C.Z as in formula (II) where L and Z are as hereinbefore defined, $R_{12}$ is hydrogen or $C_{1-6}$ alkyl, and the remaining variables are as hereinbefore defined.

Cyclisation may be effected by heating to a moderately elevated temperature in the presence of a halogenating agent and optionally in an inert solvent. A preferred halogenating agent is phosphorus oxychloride when cyclisation may be effected without solvent at the boiling point of the reaction mixture.

Alternatively the reaction may be effected by heating to a more elevated temperature optionally in an inert liquid medium such as Dowtherm or ethyl polyphosphate.

In this reaction the compound of formula (XVII) is also halogenated in situ to give a compound of formula (XVI), thus offering a convenient one-pot preparative process.

The compound of formula (XVIII) may be prepared by the conventional condensation of a compound of formula (XIX) or an alkali metal salt thereof:

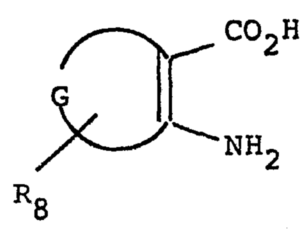

(XIX)

wherein the variables are as hereinbefore defined, with a compound of formula (XX):

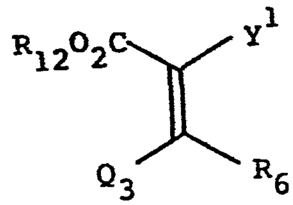

(XX)

wherein Q3 is a leaving group, for example $C_{1-6}$ alkoxy, and the remaining variables are as hereinbefore defined, with simultaneous decarboxylation.

Alternatively, compounds of the formula (XVII) may be prepared by the cyclisation in accordance with known procedures of a compound of formula (XXI):

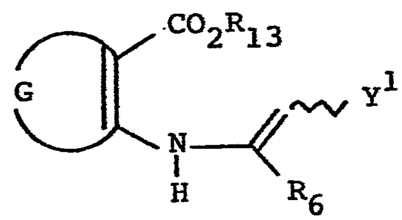

(XXI)

wherein $R_{13}$ is hydrogen or $C_{1-6}$ alkyl; and the remaining variables are as hereinbefore defined.

Cyclisation may be effected by heating in an inert solvent, such as ethanol to a slightly elevated temperature, conveniently the boiling point of the reaction mixture, in the presence of a base, such as sodium ethoxide.

The compound of formula (XXI) may be prepared by the conventional condensation of a compound of formula (XXII):

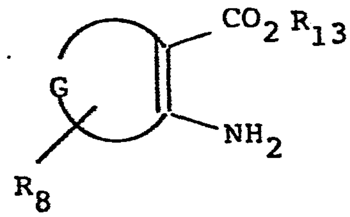

(XXII)

wherein the variables are as hereinbefore defined with a compound of formula (XXIII):

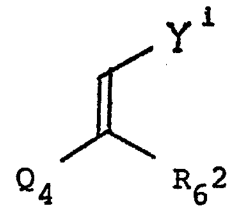

(XXIII)

wherein Q4 is a leaving group, for example $C_{1-6}$ alkoxy, and the remaining variables are as hereinbefore defined.

Compounds of formula (XVII) wherein $R_6$ is hydrogen may be prepared by base catalysed hydrolysis followed by decarboxylation, of a compound of formula (XXIV):

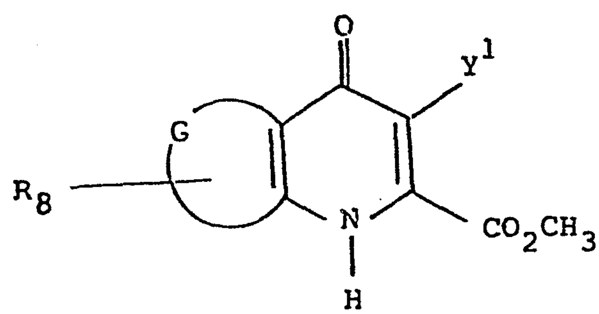

(XXIV)

The decarboxylation may occur at elevated temperatures by heating in an inert solvent such as 1,2,4- trichlorobenzene.

Compounds of the formula (XXIV) may be prepared following the procedure of J.M. Barker et al., J. Chem. Res., 1978, 4701.

Intermediates of formula (XVI) in process variant ii) a) are also of formula (I); their preparation has been described hereinbefore.

An intermediate of formula (XVI) in process variant ii) b), that is, wherein X is halo and Y is $CON(NH_2)R_1$ where $R_1$ is as hereinbefore defined may be prepared by the reaction of a compound of formula (XXV):

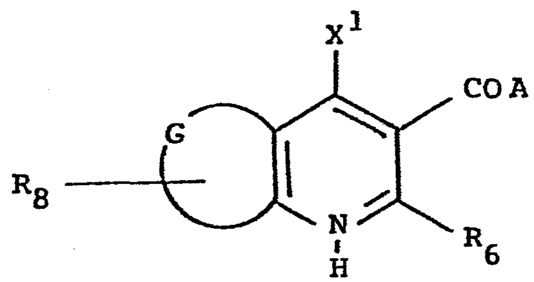

(XXV)

wherein $X^1$ is halo; A is halo; and the remaining variables are as hereinbefore defined, with a compound of formula $R_{14}NH-NH-R_1$ wherein $R_{14}$ is a deactivating N-protecting group and $R_1$ is as hereinbefore defined, followed by conventional deprotection of the product.

$R_{14}$ may be a trifluoroacetyl group and be removed subsequently by conventional base hydrolysis, or spontaneously during the cyclisation process.

In the compound of formula (XXV), $X^1$ and A will conveniently be the same halo group, in particular chloro. In this case the compound of formula (XXV) is conveniently prepared by the halogenation of a compound of formula (XVII) wherein $Y^1$ is carboxy.

An intermediate of formula (XVI) in process variant ii) c), that is , wherein X is $C_{1-6}$ alkoxyamino or azido and Y is $CONHR_1$ where $R_1$ is as hereinbefore defined may be prepared by the conventional reaction of a compound of formula (XVI) wherein X is halogen and Y is carboxyl or $C_{1-6}$ alkoxycarbonyl with an $O-C_{1-6}$ alkylhydroxylamine or an alkali metal azide, followed by conventional conversion of Y in the resultant

compound to halocarbonyl, and reaction of the latter resultant compound with an amine $R_1NH_2$ where $R_1$ is as hereinbefore defined.

Alternatively the same intermediate may be formed by the conventional reaction of a compound of formula (XXV) with an amine $R_1NH_2$ where $R_1$ is as hereinbefore defined, followed by conventional reaction of the resultant compound with an O-$C_{1-6}$ alkylhydroxylamine or an alkai metal azide.

Acid addition salts, and salts at any $COR_{10}$ carboxyl group, of a compound of formula (XII) may be formed conventionally as described hereinbefore for corresponding salts of a compound of formula (I).

The compounds of formulae (XVII) to (XXV) are known compounds or are preparable analogously to, or are routinely derivable from known compounds.

The compounds of formulae (XVI) and salts thereof are believed to be novel and as such form an aspect of the present invention.

The compounds of the present invention have anxiolytic and/or anti-depressant activity and are therefore useful in treating CNS disorders related to anxiety or depression.

The present invention also provides a pharmaceutical composition, which comprises a compound of formula (I) or a pharmaceutically acceptable salt thereof, and a pharmaceutically acceptable carrier.

The compositions may be in the form of tablets, capsules, powders, granules, lozenges, suppositories, reconstitutable powders, or liquid preparations, such as oral or sterile parenteral solutions or suspensions.

In order to obtain consistency of administration it is preferred that a composition of the invention is in the form of a unit dose.

Unit dose presentation forms for oral administration may be tablets and capsules and may contain conventional excipients such as binding agents, for example syrup, acacia, gelatin, sorbitol, tragacanth, or polyvinylpyrrolidone; fillers, for example lactose, sugar, maize-starch, calcium phosphate, sorbitol or glycine; tabletting lubricants, for example magnesium stearate; disintegrants, for example starch, polyvinylpyrrolidone, sodium starch glycollate or microcrystalline cellulose; or pharmaceutically acceptable wetting agents such as sodium lauryl sulphate.

The solid oral compositions may be prepared by conventional methods of blending, filling, tabletting or the like. Repeated blending operations may be used to distribute the active agent throughout those compositions employing large quantities of fillers. Such operations are of course conventional in the art. The tablets may be coated according to methods well known in normal pharmaceutical practice, in particular with an enteric coating.

Oral liquid preparations may be in the form of, for example, emulsions, syrups, or elixirs, or may be presented as a dry product for reconstitution with water or other suitable vehicle before use. Such liquid preparations may contain conventional additives such as suspending agents, for example sorbitol, syrup, methyl cellulose, gelatin, hydroxyethylcellulose, carboxymethylcellulose, aluminium stearate gel,hydrogenated edible fats; emulsifying agents, for

example lecithin, sorbitan monooleate, or acacia; non-aqueous vehicles (which may include edible oils), for example almond oil, fractionated coconut oil, oily esters such as esters of glycerine, propylene glycol, or ethyl alcohol; preservatives, for example methyl or propyl p-hydroxybenzoate or sorbic acid; and if desired conventional flavouring or colouring agents.

For parenteral administration, fluid unit dosage forms are prepared utilizing the compound and a sterile vehicle, and, depending on the concentration used, can be either suspended or dissolved in the vehicle. In preparing solutions the compound can be dissolved in water for injection and filter sterilized before filling into a suitable vial or ampoule and sealing. Advantageously, adjuvants such as a local anaesthetic, a preservative and buffering agents can be dissolved in the vehicle. To enhance the stability, the composition can be frozen after filling into the vial and the water removed under vacuum. Parenteral suspensions are prepared in substantially the same manner, except that the compound is suspended in the vehicle instead of being dissolved, and sterilization cannot be accomplished by filtration. The compound can be sterilized by exposure to ethylene oxide before suspending in the sterile vehicle. Advantageously, a surfactant or wetting agent is included in the composition to facilitate uniform distribution of the compound.

The compositions may contain from 0.1% to 99% by weight, preferably from 10-60% by weight, of the active material, depending on the method of administration.

The invention also provides a method of treatment of CNS disorders, in particular anxiety or depression in mammals including humans, which comprises

administering to the sufferer an anti-depressant or anxiolytic effective amount of a compound of formula (I) or a pharmaceutically acceptable salt thereof.

The dose of the compound used in the treatment of CNS disorders, such as anxiety or depression will vary in the usual way with the seriousness of the disorders, the weight of the sufferer, and the relative efficacy of the compound. However, as a general guide suitable unit doses may be 0.05 to 100 mg. for example 0.2 to 10 mg; and such unit doses may be administered more than once a day, for example two or three a day, so that the total daily dosage is in the range of about 0.01 to 10 mg/kg; and such therapy may extend for a number of weeks or months.

The invention further provides a compound of formula (I) or a pharmaceutically acceptable salt thereof, for use in the treatment of CNS disorders in particular anxiety or depression.

The following Examples illustrate the preparation of the compounds of the formula (I). The following Descriptions illustrate the preparation of intermediates to the compounds of the formula (I). All temperatures are in degrees Celsius.

The following Pharmacological Data illustrate the pharmacological activity of the compounds of formula (I).

Description 1

4,7-Dihydro-7-oxo-thieno[3,2-b]pyridine-5,6-dicarboxylic acid, dimethyl ester (D1)

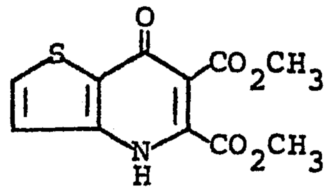

(D1)

The title compound was prepared following the procedure of J.M. Barker et al., J. Chem. Res., 1978, 4701 (81% yield; m.p. 176-178°).

Description 2

4,7-Dihydro-7-oxo-thieno[3,2-b]pyridine-5,6-dicarboxylic acid, 6-methyl ester (D2)

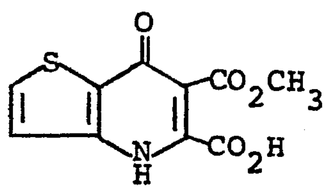

(D2)

The diester D1 (5.6g; 21mM) was added to a stirred solution of sodium hydroxide (1.76g; 44mM) in water (25ml). The mixture was kept at room temperature for 5h, then acidified with 5M hydrochloric acid (18ml). The mixture was chilled, and then filtered to give the title compound (5.18g; 98%), m.p. 162-165° (effervescence).

Nmr (DMSO) δ: 3.75 (3H,s), 7.47 (1H, d, J=6), 8.14 (1H, d, J=6).

Description 3

4,7-Dihydro-7-oxo-thieno[3,2-b]pyridine-6-carboxylic acid, methyl ester (D3)

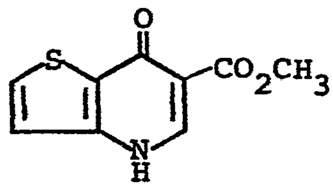

(D3)

A stirred suspension of the half-ester D2 (5.18g; 20.5mM) in 1,2,4-trichlorobenzene (70ml) was heated to 180$^{o}$, and kept at that temperature until evolution of gas ceased (approximately 10 min). The mixture was then allowed to cool to room temperature, and filtered. The brown solid obtained was washed thoroughly with petrol, and dried, to afford the title compound (3.79g; ~ 85%), frequently contaminated with the corresponding carboxylic acid. The mixture was used in further steps without purification.

Nmr (DMSO) of pure methyl ester δ: 4.01 (3H,s), 7.62 (1H, d, J=7), 7.97 (1H, d, J=7), 9.16 (1H,s).

Description 4

7-Chloro-thieno[3,2-b]pyridine-6-carboxylic acid, methyl ester (D4)

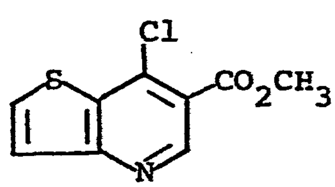

(D4)

The product from D3 (3g) was added to phosphorus oxychloride (20ml) and the solution refluxed for 4h. The solution was allowed to cool to room temperature, then

concentrated *in vacuo*. The residue was dissolved in dichloromethane (50ml) and cooled to $0^{o}$, followed by the addition of dry methanol (10ml). After stirring for 1h, water was added, and the pH adjusted to 7 using aqueous sodium carbonate. The layers were separated and the aqueous layer extracted with dichloromethane (50ml). The combined dichloromethane extracts were washed with brine, dried, and evaporated under reduced pressure to give a dark oily solid (3g). Crystallisation from chloroform/petrol afforded the title compound (2.23g; ~ 68%) as a cream solid, m.p. 95-97$^{o}$.

Nmr ($CDCl_3$) δ: 3.99 (3H,s), 7.54 (1H, d, J=5), 7.88 (1H, d, J=5), 9.08 (1H,s).

## Description 5

## 7-Chloro-thieno[3,2-b]pyridine-6-carboxylic acid, ethyl ester (D5)

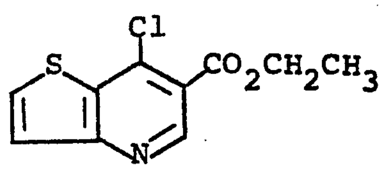

(D5)

The methyl ester prepared in Description 4 (2.20g; 9.7mM) was added to a solution of potassium t-butoxide (118mg) in dry ethanol (80ml), and the mixture stirred overnight at room temperature. The solution was then neutralised with ethanol/HCl (3 drops), and the solution concentrated under reduced pressure. The residue was extracted twice with dichloromethane (100ml) and the combined organic layers washed with brine, dried and evaporated under reduced pressure to give a yellow solid. Recrystallisation from 60-80$^{o}$ petrol afforded the title compound (1.6g; 68%) as white crystals, m.p. 79-81$^{o}$.

Nmr ($CDCl_3$) δ: 1.45 (3H, t, J=10), 4.49 (2H, q, J=10), 7.65 (1H, d, J=7), 7.97 (1H, d, J=7), 9.20 (1H,s).

Description 5

7-Chloro-thieno[3,2-b]pyridine-6-carboxylic acid, ethyl ester (D5)

Alternative Procedure

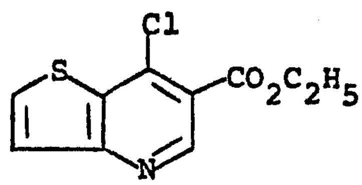

(D5)

Methyl 3-amino-thiophene-2-carboxylate (16g; 0.1mol) was added to a solution of sodium hydroxide (4.48g; 0.11mol) in water (100ml) and the mixture heated under reflux for 2hr. Evaporation in vacuo gave an off white powder (17g) which was suspended in dry toluene (250ml) and a solution of diethyl ethoxymethylenemalonate (22g; 0.1mol) in glacial acetic acid (7.5ml) was added in one portion. The stirred suspension was heated under reflux for 8hr and then allowed to cool. The mixture was partitioned between chloroform (1ℓ) and water (500ml) and the material in the organic phase gave diethyl N-(3-thienyl)aminomethyl-enemalonate as a buff solid (26.8g).

Nmr ($CDCl_3$) δ: 8.40 (1H, d, J=15Hz).

A solution of the foregoing Michael adduct (26.8g) in phosphorus oxychloride (180ml) was heated under reflux for 18hr. Work-up in a manner similar to that described in Description 4 gave a black tar which was chromatographed on Kieselgel 60 (150g) in chloroform. Combination of appropriate fractions followed by recrystallisation of the product from 60:80 petroleum ether gave the title compound as white crystals (12g; 50%), m.p. 81-82°C.

Nmr ($CDCl_3$) δ: 9.20 (1H, s).

Description 6

4,7-Dihydro-5-methyl-7-oxo-thieno[3,2-b]pyridine-6-carboxylic acid, ethyl ester (D6)

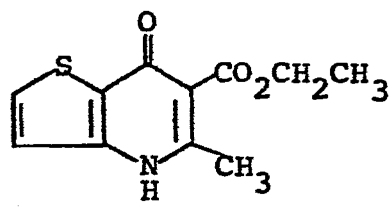

(D6)

A mixture of methyl 3-amino-thiophene-2-carboxylate (7.85g; 50mM) and ethyl 3-ethoxybut-2-enoate (15.8g; 100 mM) was heated to ~ 160° for 6h, with removal of ethanol by distillation. After cooling, the crude reaction mixture was concentrated at 80° in vacuo to afford a yellow oil (13.6g). This oil was dissolved in dry ethanol (50ml) and added dropwise to 50ml 1M sodium ethoxide in ethanol (50mM). The resultant dark red solution was refluxed for 2h, then evaporated to dryness. The residue was dissolved in water (100ml) and the solution extracted with dichloromethane (2 x 100ml). The aqueous layer was then filtered, and the pH of the filtrate adjusted to 4.5 using 5M hydrochloric acid. The mixture was chilled and then filtered to give the title compound (9.0g; 76%) as a yellow solid, m.p. 205-208° (sublimation).

Nmr (DMSO) δ: 1.27 (3H, t, J=7), 2.37 (3H, s), 4.25 (2H, q, J=7), 7.23 (1H, d, J=6), 8.02 (1H, d, J=6), 12.0-13.0 (1H, bs).

Description 7

7-Chloro-5-methyl-thieno[3,2-b]pyridine-6-carboxylic acid, ethyl ester (D7)

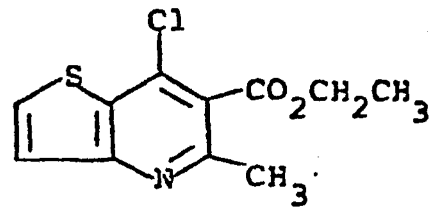

(D7)

Treatment of the pyridone prepared in D6 (6.6g; 27.9mM) with phosphorus oxychloride (40ml) using the procedure described in D4 afforded the title compound (5.2g; 73%) as a yellow oil which crystallised on standing, m.p. 115-118$^{o}$.

Nmr ($CDCl_3$) δ: 1.24 (3H, t, J=7), 2.70 (3H, s), 4.50 (2H, q, J=7), 7.53 (1H, d, J=6), 7.85 (1H, d, J=6).

Description 8

3,7-Dichloro-thieno[3,2-b]pyridine-6-carboxylic acid, ethyl ester (D8)

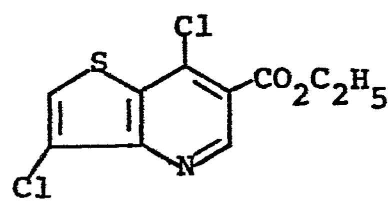

(D8)

A solution of the chloro-ester D5 (1.08g; 4.14m.mol) in sulphuryl chloride (15ml) was heated under reflux for 2hr and then evaporated to dryness. The residue was dissolved in chloroform (50ml), washed with saturated sodium hydrogen carbonate solution (3x20ml), dried ($Na_2SO_4$) and evaporation in vacuo gave a cream solid (1.1g). Chromatography on Kieselgel 60 (30g) in dichloromethane gave the title compound as colourless spars (917mg; 80%), m.p. 142-143$^o$ (from 60:80 petroleum ether).

Found: C, 42.90; H, 2.56; N, 5.02 $C_{10}H_7NO_2Cl_2$
Requires: C, 43.50; H, 2.56 and N, 5.07%

Nmr ($CDCl_3$) δ: 1.43 (3H, t, J=7.5), 4.45 (2H, q, J=7.5), 7.73 (1H,s) 9.17 (1H,s).

Description 9

4,7-Dihydro-7-oxo-5-phenyl-thieno[3,2-b]pyridine-6-carboxylic acid, ethyl ester (D9)

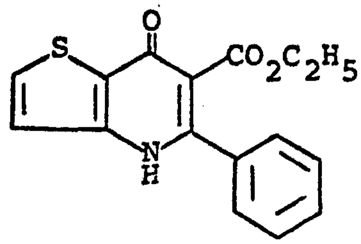

(D9)

A mixture (~ 1:1) of ethyl 3-ethoxycinnamate and ethyl 3,3-diethoxy-3-phenylpropionate (4.18g; ~ 17.8mM) was added to methyl 3-amino-thiophene-2-carboxylate (2.67g; 17mM) in xylene (80ml) containing p-toluenesulphonic acid (10mg), and refluxed vigorously for 50 minutes, with removal of ethanol by distillation. After cooling, the solution was added dropwise to 45ml 0.4M sodium ethoxide in ethanol (18mM). The resultant yellow solution was refluxed for 2h, then cooled and evaporated to dryness. The residue was dissolved in water (100ml) and the solution extracted with diethyl ether (3 x 100ml). The aqueous layer was then filtered, and the pH of the filtrate adjusted to 4.0 using 5M hydrochloric acid. The mixture was chilled, and then filtered to afford the title compound (4.1g; 81%) as a white solid, m.p. 239-242$^{o}$.

Nmr (DMSO) δ: 0.95 (3H, t, J=7), 4.00 (2H, q, J=7), 7.31 (1H, d, J=6), 7.60 (5H, s), 8.10 (1H, d, J=6), 12-13 (1H, b.s.).

Description 10

7-Chloro-5-phenyl-thieno[3,2-b]pyridine-6-carboxylic acid, ethyl ester (D10)

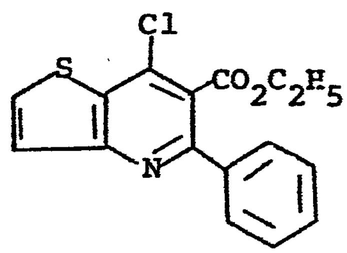

(D10)

Treatment of the pyridone prepared in D9 (3.8g; 12.7mM) with phosphorus oxychloride (40ml) using the procedure described in D4 afforded the title compound (3.75g: 93%) as a yellow oil which crystallised on standing, m.p. 70-75°.

Nmr ($CDCl_3$) δ: 1.10 (3H, t, J=7), 4.24 (2H, q, J=7), 7.30-7.80 (6H, m), 7.93 (1H, d, J=6).

## Description 11

## 4-Chloro-2-ethyl-thieno[2,3-b]pyridine-5-carboxylic acid, ethyl ester (D11)

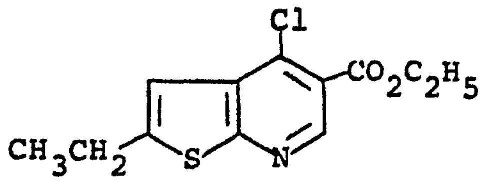

(D11)

A mixture of 2-amino-5-ethyl-thiophene-3-carboxylic acid (10g; 54mM) and diethyl ethoxymethylenemalonate (11.67g; 54mM) in toluene (150ml) was refluxed for 6h, under nitrogen. The dark brown solution was cooled then evaporated to dryness. The resultant black oil was chromatographed on silica (500g) using 70% chloroform-petrol as eluent. Combination of appropriate fractions afforded diethyl N-(5-ethyl-2-thienyl)-aminomethylene-malonate as a yellow oil (9.5g).

Nmr ($CDCl_3$) δ: 1.25 (9H,m), 2.66 (2H,q,J=7), 4.18 (4H,m), 6.35 (2H, b.s.), 8.10 (1H,d, J=14)

A solution of the foregoing Michael adduct (9.5g) in phosphorus oxychloride (53ml) was heated under reflux for 4h. Work-up in a manner similar to that described in Description 5 - alternative procedure afforded the title compound as a yellow solid (4g; 42%), m.p. 37-41$^{o}$.

Nmr ($CDCl_3$): 1.40 (3H,t,J=7), 1.42 (3H,t,J=6), 2.95 (2H,q, J=6), 4.42 (2H,q,J=7), 7.18 (1H, b.s.), 8.90 (1H,s).

Description '12

4-Chloro-2-ethyl-6-methyl-thieno[2,3-b]pyridine-5-carboxylic acid, ethyl ester (D12)

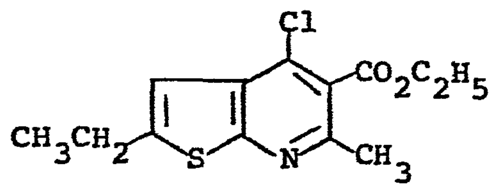

(D12)

The title compound was prepared from ethyl 2-amino-5-ethylthiophene-3-carboxylate and ethyl 3-ethoxybut-2-enoate using a method similar to that described in Description 6 and Description 4. m.p. 38-40°.

Nmr ($CDCl_3$) δ: 1.43 (3H,t,J=7), 1.46 (3H,t,J=7), 2.68 (3H,s), 2.98 (2H, b.q., J=7), 4.51 (2H, q, J=7), 7.10 (1H,t,J=1).

Description 13

4-Chloro-thieno[3,4-b]pyridine-3-carboxylic acid, methyl ester (D13)

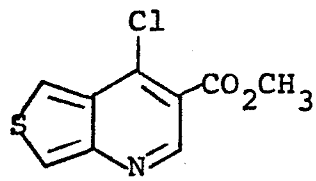

(D13)

Methyl 3-amino-thiophene-4-carboxylate was converted into 1,4-dihydro-4-oxo-thieno[3,4-b]pyridine-2,3-dicarboxylic acid, dimethyl ester using a procedure similar to that described in Liebigs Ann. Chem., 1976 1972-1981. Conversion into the title compound was achieved using a procedure similar to that of Descriptions 2,3 and 4.

Nmr ($CDCl_3$) δ: 3.90 (3H,s), 7.93 (1H,d,J=4), 8.05 (1H,d, J=4), 8.90 (1H,s).

Description 14

4-Chloro-3,6-dimethyl-thieno[2,3-b]pyridine-5-carboxylic acid, ethyl ester (D14)

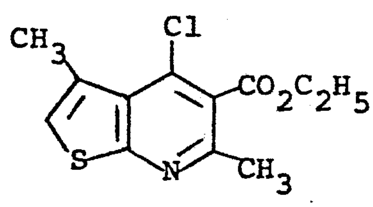

(D14)

Prepared by strict analogy to Description 12.
m.p. 67-68.5°.

Nmr ($CDCl_3$) δ: 1.43 (3H,t,J=7), 2.62 (3H,s), 2.66 (3H, d,J=1), 4.47 (2H,q,J=7), 7.12 (1H,d,J=1).

Description 15

7-Chloro-2-methyl-thieno[3,2-b]pyridine-6-carboxylic acid, ethyl ester (D15)

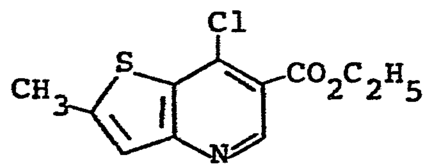

(D15)

Prepared by strict analogy to Description 5 - alternative procedure.

Nmr ($CDCl_3$) δ: 1.40 (3H,t), 2.63 (3H,s), 4.37 (2H,q), 7.13 (1H,s), 8.93 (1H,s).

Example 1

7-(2-Phenylhydrazino)-thieno[3,2-b]pyridine-6-carboxylic acid, ethyl ester, monohydrochloride (E1)

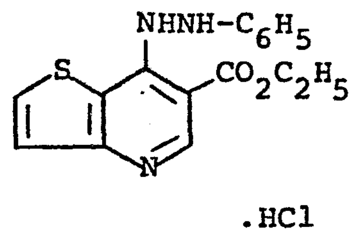

(E1)

A solution of the ester (D5) (1.11g; 4.61mM) in dry ethanol (36ml) containing phenylhydrazine (0.498g; 4.61 mM) was refluxed under nitrogen for 18h. The solution was then evaporated to dryness, and the residue was crystallised twice from ethanol/petrol. Recrystallisation from ethanol afforded the title compound (0.59g; 37%) as pale yellow needles, m.p. 140$^{o}$ (softening).

Nmr (DMSO) δ: 1.35 (3H, t, J=7), 4.38 (2H, q, J=7), 6.75-7.35 (5H, m), 7.60 (1H, d, J=6), 8.35 (1H, d, J=6), 8.88 (1H,s), 8.95 (1H,s), 10.83 (1H,s).

| | |
|---|---|
| Found $M^+$ | 313.0886 |
| $C_{16}H_{15}N_3O_2S$ requires | 313.0885 |

Example 2

7-(4-Chlorophenylhydrazino)-thieno[3,2-b]pyridine-6-carboxylic acid, ethyl ester, monohydrochloride (E2)

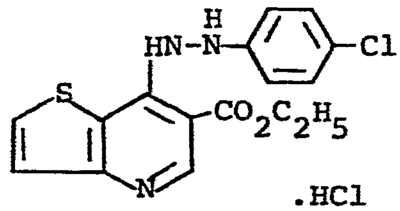

.HCl (E2)

A solution of the ester (D5) (900mg; 3.72mmol) and 4-chlorophenylhydrazine (532mg; 3.72mmol) in ethanol (30ml) was treated in a similar manner to that described in Example 1 to give the title compound as cream needles (552mg; 39%). m.p. 165-168$^{o}$ (from ethanol).

Nmr (DMSO) δ: 1.40 (3H, t, J=7), 4.25-4.60 (2H, q, J=7), 6.95 and 7.33 (4H, ABq, J=8.5); 7.17 and 7.66 (2H, ABq, J=6); 9.01 (1H,s); 9.15 and 10.93 (2 x 1H,s, ex $D_2O$).

Found $M^+$ 347.0494

$C_{16}H_{14}N_3O_2SCl$ requires 347.0495

Example 3

7-(2-(4-Nitrophenyl)hydrazino)-thieno[3,2-b]pyridine-6-carboxylic acid, ethyl ester, monohydrochloride (E3)

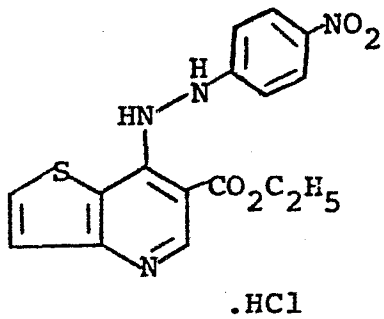

(E3)

A solution of the ester D5 (1.00g; 4.14m.mol) and 4-nitrophenylhydrazine (634mg; 4.1m.mol) in ethanol (30ml) was treated in a manner similar to that described in Example 1 to give the title compound as pale orange crystals (822mg; 50%), m.p. 211-213°C (dec).

Found: C, 48.93; H, 3.89; N, 14.32 $C_{16}H_{15}N_4O_4SCl$

Requires: C, 48.67; H, 3.83 and N, 14.19%

Nmr ($d^6$DMSO) δ: 1.40 (3H, t, J=7), 4.45 (2H, q, J=7), 7.10, 8.18 (4H, ABq, J=8), 7.67, 8.46 (2H, ABq, J=6), 9.05 (1H, s).

Example 4

7-(2-(4-Isopropylphenyl)hydrazino)-thieno[3,2-b]pyridine -6-carboxylic acid, ethyl ester, monohydrochloride (E4)

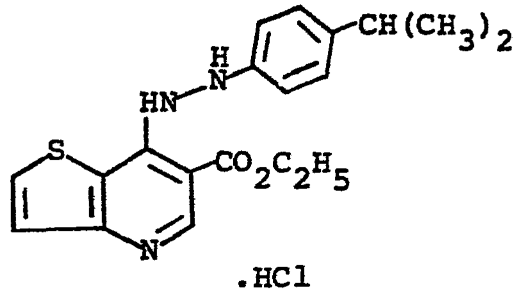

(E4)

A solution of the ester D5 (1.00g; 4.14m.mol) and 4-isopropylphenylhydrazine (621mg; 4.14m.mol) in ethanol (30ml) was treated in a similar manner to that in Example 1 to give the title compound as yellow needles (408mg; 25%), m.p. 207-210°.

Found: C, 58.50; H, 5.67; N, 11.03 $C_{19}H_{22}N_3O_2SCl$

Requires: C, 58.23; H, 5.66 and N, 10.72%.

Nmr ($d^6$DMSO) δ: 1.16 (6H, d, J=7), 1.40 (3H, t, J=7), 2.55-3.00 (1H,m); 4.43 (2H, q, J=7), 6.84, 7.15 (4H, ABq, J=8), 7.60, 8.37 (2H, ABq, J=6), 9.04 (1H,s).

Example 5

7-(2-(4-Methylphenyl)hydrazino)-thieno[3,2-b]pyridine-6-carboxylic acid, ethyl ester, monohydrochloride (E5)

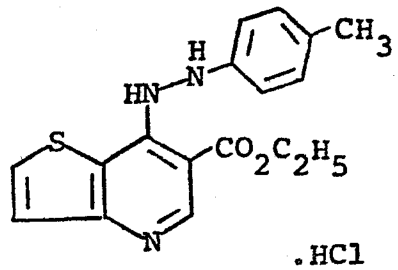

.HCl (E5)

The chloro-ester D5 (2.20g; 9.11m.mol) and 4-methylphenyl-hydrazine (1.14g; 9.3m.mol) in ethanol (40ml) were treated in a manner similar to that in Example 1 to give the title compound as white crystals (910mg; 27%), m.p. 204-206°.

Found: C, 56.37; H, 4.85; N, 11.84 $C_{17}H_{18}N_3O_2SCl$

Requires: C, 56.12; H, 4.99 and N, 11.55%.

Nmr ($d^6$DMSO) δ: 1.40 (3H, t, J=7), 2.22 (3H,s), 4.42 (2H, q, J=7), 6.80, 7.07 (4H, ABq, J=8.5), 7.63, 8.38 (2H, ABq, J=6), 9.00 (1H,s).

Example 6

7-(2-(2-Pyridyl)hydrazino)-thieno[3,2-b]pyridine-6-carboxylic acid, ethyl ester, monohydrochloride (E6)

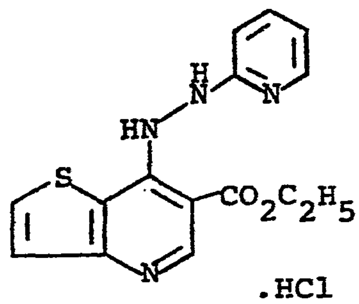

(E6)

The chloro-ester D5 (1.07g; 4.42m.mol) and 2-hydrazino-pyridine (483mg; 4.42m.mol) in ethanol (30ml) were treated in a similar manner to that described in Example 1 to give the title compound as pale yellow crystals (1.21g; 65%), m.p. 205-207°.

Found: C, 50.33; H, 4.41; N, 15.55 $C_{15}H_{15}N_4O_2SCl.\frac{1}{2}H_2O$

Requires: C, 50.08; H, 4.48 and N, 15.57%

Nmr ($d^6$DMSO) δ: 1.40 (3H,t,J=7), 4.25-4.55 (2H,q,J=7), 6.85-7.05 (2H,m), 7.61, 8.37 (2H, ABq, J=6), 7.80 (1H,m), 9.05 (1H,s).

Example 7

7-(2-(3,5-Dichlorophenyl)hydrazino)-thieno[3,2-b]pyridine-6-carboxylic acid, ethyl ester, monohydrochloride (E7)

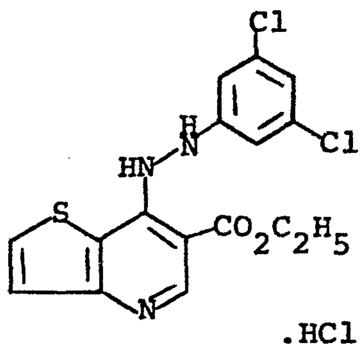

(E7)

The chloro-ester D5 (2.18g; 9.03m.mol) and 3,5-dichlorophenylhydrazine (1.60g; 9.03m.mol) in ethanol (50ml) were treated in a similar manner to that described in Example 1 to give the title compound as cream needles (2.65g; 70%), m.p. 220-222° (effervescence).

Found: C, 45.77; H, 3.42; N, 9.81 $C_{16}H_{14}N_3O_2SCl_3$

Requires: C, 45.89; H, 3.37 and N, 10.04%

Nmr ($d^6$DMSO) δ: 1.38 (3H, t, J=7), 4.25-4.60 (2H,q,J=7), 6.95 (2H,d,J=2), 7.03 (1H,t,J=2), 7.63, 8.40 (2H, ABq, J=6), 9.00 (1H,s).

Example 8.

7-(2-(4-Methoxyphenyl)hydrazino)-thieno[3,2-b]pyridine-6-carboxylic acid, ethyl ester, monohydrochloride (E8)

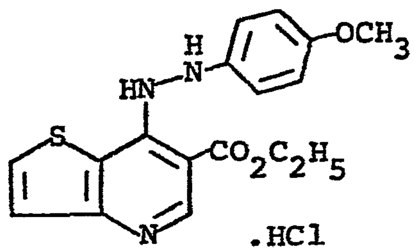

.HCl (E8)

The chloro-ester D5 (1.35g; 5.60m.mol) and 4-methoxyphenylhydrazine (800mg; 5.76m.mol) in ethanol (30ml) were treated in a manner similar to that described in Example 1 to give the title compound as yellow needles (720mg; 34%), m.p. 148-152°.

Found: C, 52.56; H, 5.05; N, 10.40; Cl, 9.02

$C_{17}H_{18}N_3O_3SCl.\frac{1}{2}H_2O$.

Requires: C, 52.21; H, 4.92; N, 10.80 and Cl, 9.12%.

Nmr ($d^6$DMSO) δ: 1.38 (3H,t,J=7.5), 3.70 (3H,s), 4.43 (2H,q,J=7.5), 6.86 (4H,s), 7.65, 8.40 (2H, ABq, J=6), 9.01 (1H,s).

Example 9.

3-Chloro-7-(2-phenylhydrazino)-thieno[3,2-b]pyridine-6-carboxylic acid, ethyl ester, monohydrochloride (E9)

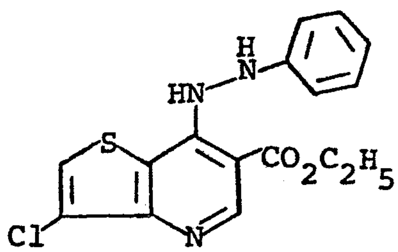

(E9)

.HCl

The dichloro-ester D8 (460mg; 1.67m.mol) and phenylhydrazine (180mg; 1.67m.mol) in ethanol (15ml) were treated in a manner similar to that in Example 1 to give the title compound as yellow crystals (370mg; 58%), m.p. 202-205°.

Found: C, 50.31; H, 3.90; N, 10.65 $C_{16}H_{15}N_3O_2SCl$

Requires: C, 50.01; H, 3.93 and N, 10.93%

Nmr ($d^6$DMSO) δ: 1.40 (3H,t,J=7), 4.40 (2H,q,J=7), 6.75-7.40 (5H,m), 8.32 (1H,s), 8.87 (1H,s).

Example 10

5-Methyl-7-(2-phenylhydrazino)-thieno[3,2-b]pyridine-6-carboxylic acid, ethyl ester, monohydrochloride (E10)

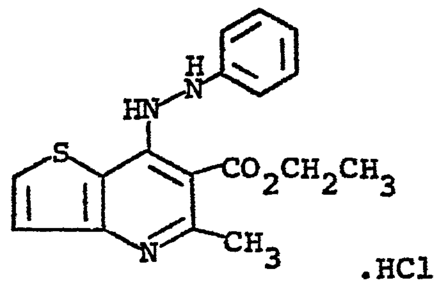

.HCl

(E10)

The chloro-ester D7 (1.28g; 5mM) and phenylhydrazine (0.54g; 5mM) in ethanol (15ml) were treated in a manner similar to that in Example 1 to give the title compound as white needles (0.75g; 41%), m.p. 215-216° (decomposition; softening from 185°).

Found: C, 56.56; H, 4.94; N, 11.73 $C_{17}H_{18}N_3O_2SCl$

Requires: C, 56.12; H, 4.99; N, 11.55%

Nmr (DMSO) δ: 1.30 (3H, t, J=7), 2.73 (3H,s) 4.37 (2H, q,J=7), 6.70-7.30 (5H, m, overlapping signals), 7.54 (1H,d,J=6), 8.33 (1H,d,J=6), 8.90 (1H,s, ex $D_2O$), 10.60 (1H,s, ex $D_2O$).

**Example 11**

**7-(2-(4-Chlorophenyl)hydrazino)-5-methyl-thieno[3,2-b]pyridine-6-carboxylic acid, ethyl ester, monohydrochloride (E11)**

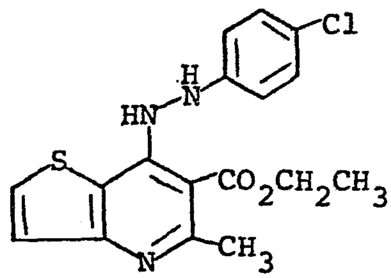

.HCl (E11)

The chloro-ester D7 (1.28g; 5mM) and 4-chlorophenylhydrazine (0.712g; 5mM) in ethanol (25ml) were treated in a manner similar to that in Example 1 to give the title compound as white needles (0.78g; 40%), m.p. 215-217° (decomposition).

Found: C, 50.64; H, 4.18; N, 10.37 $C_{17}H_{17}N_3O_2SCl_2$

Requires: C, 51.26; H, 4.30; N, 10.55%

Found $M^+$ 361.06506 $C_{17}H_{16}N_3O_2Cl$

Requires: 361.06516

Nmr (DMSO) δ: 1.35 (3H,t,J=7), 2.76 (3H,s), 4.42 (2H, q, J=7), 6.91 (2H,d,J=9), 7.35 (2H,d,J=9), 7.61 (1H,d,J=6), 8.36 (1H,d,J=6), 9.11 (1H,s, ex $D_2O$), 10.60 (1H,s, ex $D_2O$).

Example 12

5-Phenyl-7-(2-phenylhydrazino)-thieno[3,2-b]pyridine-6-carboxylic acid, ethyl ester, monohydrochloride (E12)

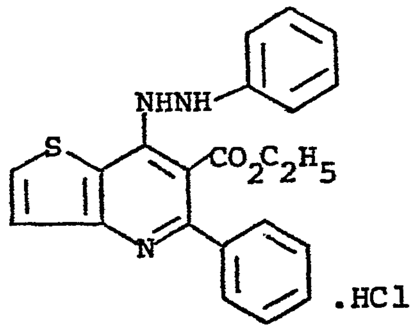

(E12)

The chloro-ester D10 (1.59g; 5mM) and phenylhydrazine (0.54g; 5mM) in ethanol (30ml) were treated in a manner similar to that described in Example 1 to give the title compound as pale yellow crystals (0.7g; 32%), m.p. 218-220° (dec).

Found: C, 61.78; H, 4.78; N, 9.81. $C_{22}H_{20}N_3O_2SCl$

Requires: C, 62.04; H, 4.73; N, 9.87%

Found $M^+$ 389.1190

$C_{22}H_{19}N_3O_2S$ requires: 389.1198

Nmr (DMSO) δ: 0.90 (3H, t, J=7), 4.07 (2H, q, J=7), 6.50-7.50 (5H, m), 7.65 (5H, s), 7.70 (1H, d, J=6), 8.42 (1H, d, J=6), 9.00 (1H, s, ex. $D_2O$), 10.60 (1H, s, ex. $D_2O$).

Example 13

7-(2-(4-Chlorophenyl)hydrazino)-5-phenyl-thieno[3,2-b] pyridine-6-carboxylic acid, ethyl ester, monohydrochloride (E13)

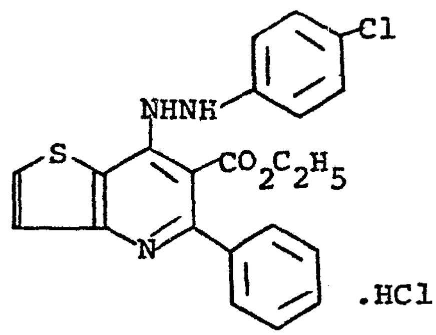

(E13)

The chloro-ester D10 (3.05g; 9.6mM) and 4-chlorophenylhydrazine (1.38g; 9.6mM) in ethanol (30ml) were treated in a manner similar to that described in Example 1 to give the title compound as a white solid (3.4g; 77%), m.p. 218-220° (dec.).

Found: C, 57.45; H, 4.15; N, 9.12. $C_{22}H_{19}N_3O_2SCl_2$
Requires: C, 57.39; H, 4.16; N, 9.13%

Found $M^+$ 423.0814

$C_{22}H_{18}N_3O_2SCl$ requires 423.0808

Nmr (DMSO) δ: 0.87 (3H, t, J=7), 4.04 (2H, q, J=7), 6.92 (2H, d, J=9), 7.32 (2H, d, J=9), 7.62 (5H,s), 7.65 (1H, d, J=6), 8.38 (1H, d, J=6), 9.18 (1H, s, ex. $D_2O$), 10.58 (1H, s, ex. $D_2O$).

Example 14

3-Chloro-7-(2-(4-chlorophenyl)hydrazino)-thieno[3,2-b] pyridine-6-carboxylic acid, ethyl ester, monohydrochloride (E14)

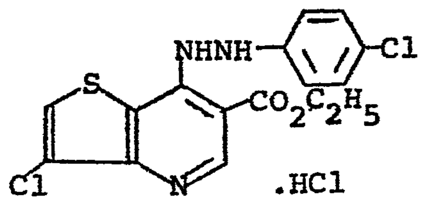

(E14)

The title compound was prepared in 75% yield from the dichloroester D8 and 4-chlorophenylhydrazine using a method similar to Example 1. m.p. 200°C (dec.).

Found $M^+$ 381.0103

$C_{16}H_{13}N_3O_2Cl_2S$ requires 381.0105

Nmr (DMSO-$d_6$) δ: 1.45 (3H, t, J=6Hz); 4.48 (2H, q, J=6Hz); 6.96 (2H, d, J=9Hz); 7.37 (2H, d, J=9Hz); 8.37 (1H, s); 8.95 (1H, s, ex $D_2O$); 8.96 (1H, s); 10.60 (1H, s, ex $D_2O$).

Example 15

4-(2-(4-Chlorophenyl)hydrazino)-thieno[2,3-b]pyridine-5-carboxylic acid, ethyl ester, monohydrochloride (E15)

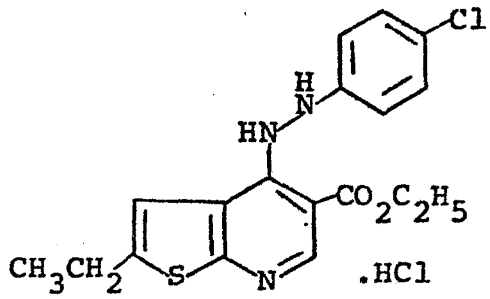

(E15)

The chloro-ester D11 (1.88g; 7mM) and 4-chlorophenylhydrazine (0.99g; 7mM) in ethanol (15ml) were treated in a manner similar to that described in Example 1 to give the title compound as white needles (0.7g; 25%), m.p. 188-189° (dec.).

Found: C, 52.22; H, 4.68; N, 9.99. $C_{18}H_{19}N_3O_2SCl_2$

Requires: C, 52.43; H, 4.64; N, 10.19%.

Found $M^+$ 375.0801. $C_{18}H_{18}N_3O_2SCl$

Requires: 375.0808

Nmr (DMSO) δ: 1.18 (3H,t,J=7), 1.32 (3H,t,J=7), 2.82 (2H,q,J=7), 4.33 (2H,q,J=7), 6.85 (2H, b.d., J=8), 7.27 (2H, b.d., J=8), 7.70 (1H,s), 8.74 (1H, b.s., ex. $D_2O$), 8.85 (1H,s).

The following examples E16 to E19 were prepared by strict analogy to Example 15.

0126970

Example 16

7-(2-(4-Chlorophenyl)hydrazino)-2-methyl-thieno[3,2-b] pyridine-6-carboxylic acid, ethyl ester, monohydrochloride (E16)

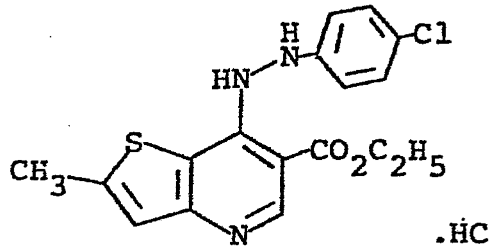

(E16)

.HCl

Nmr ($d_6$DMSO) δ: 1.40 (3H,t), 2.53 (3H,s), 4.45 (2H,q), 6.95, 7.35 (4H,ABq,J=8), 7.42 (1H,s), 8.95 (1H,s,ex $D_2O$), 9.00 (1H,s), 10.80 (1H,s,ex $D_2O$).

Example 17

2-Methyl-7-(2-phenylhydrazino)-thieno[3,2-b]pyridine-6-carboxylic acid, ethyl ester, monohydrochloride (E17)

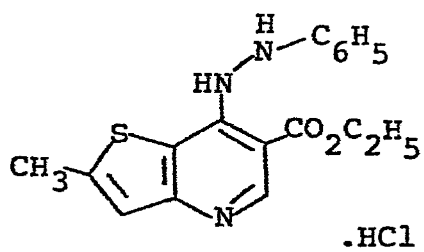

.HCl (E17)

Example 18

4-(2-(4-Chlorophenyl)hydrazino)-thieno[3,4-b]pyridine-3-carboxylic acid, methyl ester, monohydrochloride (E18)

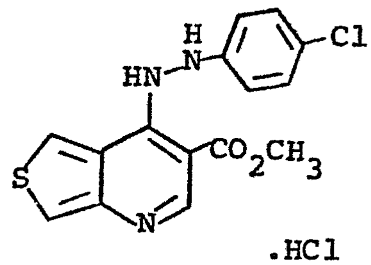

.HCl (E18)

m.p. 177-180°.

Found: C, 46.67; H, 3.57; N, 10.89 $M^+$ 333.0343 $C_{15}H_{13}N_3O_2SCl_2$
Requires: C, 48.66; H, 3.54; N, 11.35% and 333.0339.

Nmr ($d_6$ DMSO) δ: 3.94 (3H,s), 7.00, 7.36 (4H, ABq, J=10), 8.11 (1H,d,4), 8.96 (1H,s), 9.34 (1H,d, J=4), 9.36 (1H,s, ex $D_2O$), 12.40 (1H,br, ex $D_2O$).

Example 19

2,5-Dihydro-2-phenyl-3H-pyrazolo[3,4-d]thieno[3,2-b] -3-one (E19)

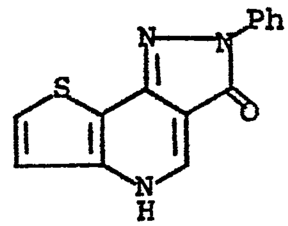

(E19)

A solution of the ester prepared in Description 5 (1.53g; 6.34mM) in dry ethanol (50ml) containing phenylhydrazine (0.68g, 6.33mM) was refluxed under nitrogen for 24h. Xylene (50ml) was added, and the ethanol was distilled out of the reaction mixture. The xylene solution was then refluxed under nitrogen for 7 days and then allowed to cool to room temperature. Filtration afforded, after drying *in vacuo*, a brown solid (0.8g). Purification was effected by dissolving the solid in the minimum volume of 10% aqueous sodium hydroxide, and washing with diethyl ether. The aqueous layer was then filtered and the pH adjusted to ~8 using aqueous ammonium chloride. The precipitate formed was filtered, and washed successively with cold water, cold methanol and diethyl ether to yield the title compound as a brown solid (0.40g; 24%), m.p. ~220$^{o}$ (decomposition).

Nmr (DMSO) δ: 7.0-8.4 (7H, m, overlapping signals),
8.75 (1H,s).

Found $M^+$ 267.0461

$C_{14}H_9N_3OS$ requires 267.0466

Example 20

2-(4-Chlorophenyl)-2,5-dihydro-4-methyl-3H-pyrazolo [3,4-d]thieno[3,2-b]pyridin-3-one (E20)

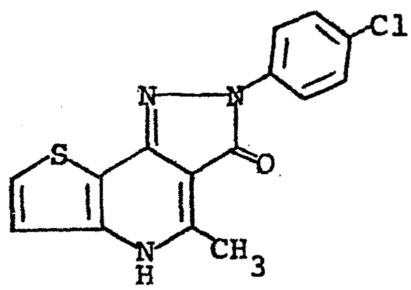

(E20)

A suspension of the hydrazino ester prepared in Example 11 (2.4g; 6.03mM) in Dowtherm A (46ml) was heated to 180° for 5h, under nitrogen. The mixture was then cooled, and diluted with an equal volume of petroleum ether. Filtration afforded the crude product as a yellow solid. Purification was effected by dissolving the solid in water (150ml) containing 10% aqueous sodium hydroxide (4ml) and dimethylformamide (4ml), and washing with diethyl ether (x3). The aqueous layer was then filtered, and the pH adjusted to ~ 8 using aqueous ammonium chloride. The resulting precipitate was filtered, washed with water, then dried at 50° *in vacuo* to yield the title compound as a light yellow solid (1.7g; 89%), m.p. 321-325° (decomposition).

Nmr (DMSO) δ: 2.83 (3H, s), 7.37 (1H, d, J=6), 7.49 (2H, d, J=9), 8.01 (1H, d, J=6), 8.28 (2H, d, J=9).

Found $M^+$ 315.0234

$C_{15}H_{10}N_3OSCl$ requires 315.0233

Example 21

2-(4-Chlorophenyl)-2,5-dihydro-3H-pyrazolo[3,4-d]thieno[3,2-b]pyridin-3-one (E21)

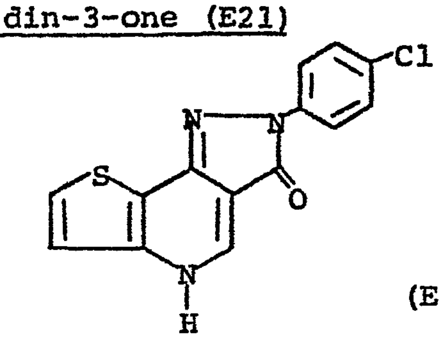

(E21)

The title compound was prepared in 30% yield from the chloro-ester (D5) and 4-chlorophenylhydrazine in a manner similar to that employed in Examples 1 and 20.

m.p. 336-340°C

Found: C, 53.07; H, 2.91; N, 13.10 $C_{14}H_8N_3OSCl.H_2O$

Requires: C, 52.60; H, 3.15 and N, 13.14%

Found: $M^+$ 301.0076 Calc. for $C_{14}H_8N_3OSCl$ 301.0076

Nmr (DMSO) δ: 7.46 (1H, d, J6Hz), 7.53 (2H, d, J9Hz), 8.07 (1H, d, J6Hz), 8.30 (2H, d, J9Hz), 8.77 (1H,s) and 11-14 (1H, br, ex $D_2O$).

Example 22

2-(3,5-Dichlorophenyl)-2,5-dihydro-3H-pyrazolo[3,4-d] thieno[3,2-b]pyridin-3-one (E22)

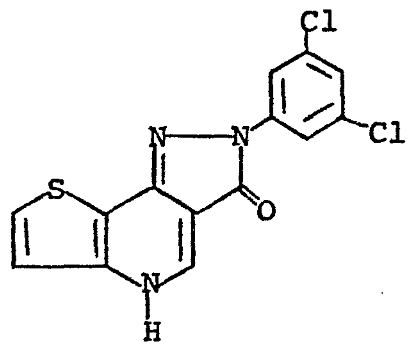

(E22)

The title compound was prepared from the chloro-ester D5 and 3,5-dichlorophenylhydrazine in 47% yield using a method similar to that described in Examples 1 and 20. m.p. 327-331$^{o}$.

Found: C, 49.04; H, 2.20; N, 12.10 $C_{14}H_7N_3OSCl_2$

Requires: C, 50.00; H, 2.10 and N, 12.50%

Found $M^+$ 334.9699 Calc. for $C_{14}H_7N_3OSCl_2$ 334.9687

Nmr ($d^6$DMSO) δ: 7.32 (1H,t,J=2); 7.41, 8.05 (2H,ABq,J=6), 8.27 (2H,d,J=2), 8.77 (1H,s).

Example 23

2,5-Dihydro-2-(4-methylphenyl)-3H-pyrazolo[3,4-d]thieno[3,2-b]pyridin-3-one (E23)

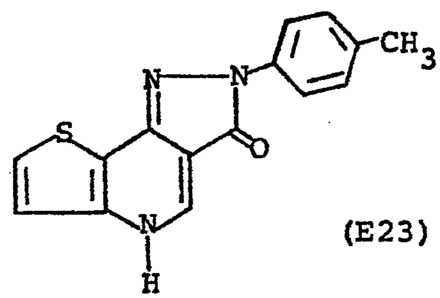

(E23)

The title compound was prepared from the chloro-ester D5 and 4-methylphenylhydrazine in 55% yield using a method similar to that described in Examples 1 and 20. m.p. 320-323°.

Found: C, 63.53; H, 3.94; N, 14.62 $C_{15}H_{11}N_3OS$

Requires: C, 64.04; H, 3.94 and N, 14.94%

Nmr ($d^6$DMSO) δ: 2.33 (3H,s), 7.25, 8.10 (4H,ABq,J=8), 7.45, 8.05 (2H,ABq,J=6), 8.71 (1H,s).

Example 24

2,5-Dihydro-2-(4-methoxyphenyl)-3H-pyrazolo[3,4-d]thieno [3,2-b]pyridin-3-one (E24)

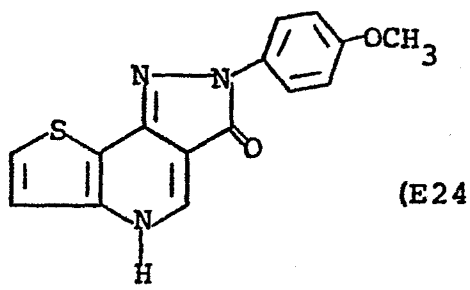

(E24)

The title compound was prepared in 60% yield from the chloro-ester D5 and 4-methoxyphenylhydrazine using a method similar to that described in Examples 1 and 20. For the cyclisation step two equivalents of potassium carbonate as base was used in ethanol at reflux.
m.p. 330-332$^{o}$ (dec).

Found: C, 60.09; H, 3.60; N, 14.04 $C_{15}H_{11}N_3O_2S$
Requires: C, 60.59; H, 3.73 and N, 14.13%

Nmr ($d^6$DMSO): δ: 3.80 (3H, s), 7.02, 8.10 (4H, ABq, J=8), 7.43, 8.05 (2H, ABq, J=6), 8.67 (1H, s).

Example 25

2,5-Dihydro-4-methyl-2-phenyl-3H-pyrazolo[3,4-d]thieno [3,2-b]pyridin-3-one (E25)

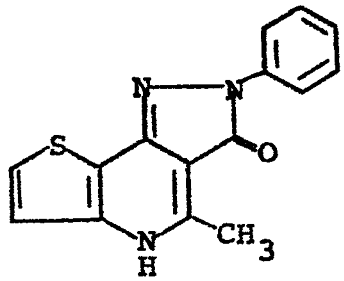

(E25)

The title compound was prepared from the chloro-ester D7 and phenylhydrazine in 53% yield using a method similar to that described in Examples 1 and 20.
m.p. 294-298°.

Found: C, 60.05; H, 4.24; N, 14.03 $C_{15}H_{11}N_3OS.H_2O$

Requires: C, 60.18; H, 4.38; N, 14.04%

Found $M^+$ 281.0626 $C_{15}H_{11}N_3OS$

Requires 281.0623

Nmr (DMSO) δ: 2.80 (3H,s), 7.0-7.6 (4H,m,overlapping signals), 7.96 (1H,d,J=6), 8.22 (2H, d, J=9).

Example 26

6-Chloro-2-(4-chlorophenyl)-2,5-dihydro-3H-pyrazolo[3,4-d]-thieno[3,2-b]pyridin-3-one (E26)

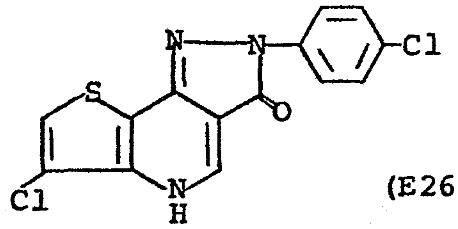

(E26)

The title compound was prepared in 60% yield from the dichloroester D8 and 4-chlorophenylhydrazine using a method similar to that described in Examples 1 and 20. For the cyclisation step, two equivalents of potassium carbonate as base was used in sec butanol at reflux, for 18hrs, under an inert atmosphere. m.p. > 300°C.

Found $M^+$ 334.9682

$C_{14}H_7N_3OSCl_2$ requires: 334.9686

Nmr (DMSO-$d_6$) δ: 7.48 (2H, d, J=9Hz); 8.12 (1H, s); 8.27 (2H, d, J=9Hz); 8.55 (1H, s).

Example 27

2-(4-Chlorophenyl)-2,5-dihydro-4,8-dimethyl-3H-pyrazolo [3,4-d]thieno[2,3-b]pyridin-3-one (E27)

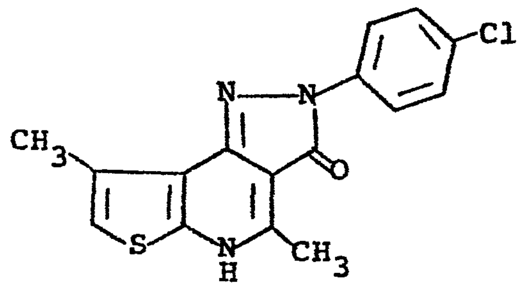

(E27)

A solution of the chloroester D14 (3.77g; 14mM) in dry ethanol (70ml) containing p-chlorophenylhydrazine (3.98g; 28mM) was refluxed under nitrogen for 67h, then cooled to room temperature. Filtration afforded the crude product as a yellow solid (3g). Purification was effected by dissolving the solid in 10% aqueous sodium hydroxide (10ml) and dimethylformamide (10ml) followed by addition of water (200ml). The solution was then washed with diethyl ether (3 x 100ml) and filtered. The pH of the filtrate was adjusted to 8 using saturated aqueous ammonium chloride. The precipitate was filtered, washed successively with cold water, cold ethanol and diethyl ether, then dried *in vacuo* to afford the title compound (2.7g; 59%) as a yellow solid, m.p. > 346°.

Nmr (DMSO) δ: 2.6 (3H, d, J = 1), 2.75 (3H, s), 7.12 (1H, d, J = 1), 7.45 (2H, d, J = 8), 8.27 (2H, d, J = 8).

Found $M^+$: 329.0381

$C_{16}H_{12}N_3OSCl$ requires: 329.0389

Example 28

2-(4-Chlorophenyl)-2,5-dihydro-7-ethyl-3H-pyrazolo[3,4-d]thieno[2,3-b]pyridin-3-one (E28)

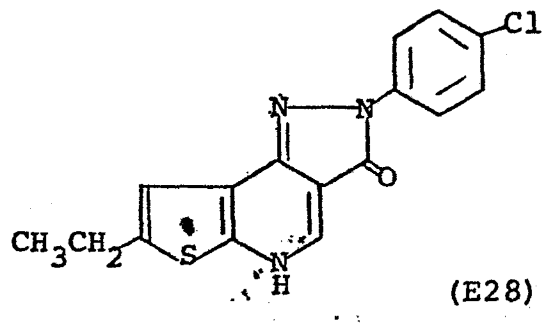

(E28)

The title compound was prepared from the chloro-ester D11 and 4-chlorophenylhydrazine in 31% yield using a method similar to that described in Example 27. m.p. 295-300$^{o}$ (dec.).

Found: C, 55.95; H, 3.62; N, 12.24. $C_{16}H_{12}N_3SOCl$

Requires: C, 55.25; H, 3.48; N, 12.08%.

Found $M^+$ 329.0388. $C_{16}H_{12}N_3SOCl$

Requires: 329.0389

Nmr (DMSO) δ: 1.35 (3H,t,J=7), 2.96 (2H,d,J=7), 7.34 (1H, b.s.), 7.50 (2H, b.d., J=8), 8.26 (2H, b.d., J=8), 8.70 (1H,s).

Example 29

2-(4-Chlorophenyl)-2,5-dihydro-7-ethyl-4-methyl-3H-pyrazolo[3,4-d]thieno[2,3-b]pyridin-3-one (E29)

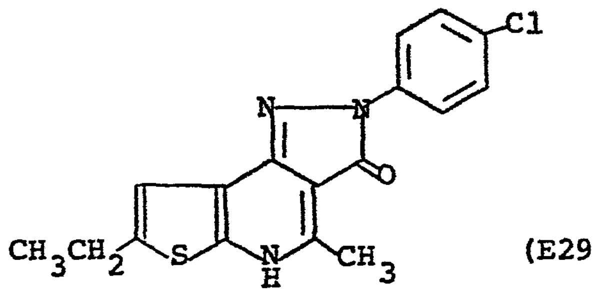

(E29)

The title compound was prepared from the chloro-ester D12 and 4-chlorophenylhydrazine in 64% yield using a method similar to that described in Example 27. m.p. 320-321°.

Found: C, 59.11; H, 4.16; N, 12.12. $C_{17}H_{14}N_3SOCl$
Requires: C, 59.38; H, 4.10; N, 12.22%.

Found $M^+$ 343.0547. $C_{17}H_{14}N_3SOCl$
Requires: 343.0546

Nmr (DMSO) δ: 1.35 (3H,t,J=7), 2.81 (3H,s), 2.93 (2H, b.q., J=7), 7.30 (1H, b.s.), 7.51 (2H, b.d., J=9), 8.30 (2H, b.d., J=9).

Example 30

2,5-Dihydro-2-(2-pyridyl)-3H-pyrazolo[3,4-d]thieno[3,2-b] pyridin-3-one (E30)

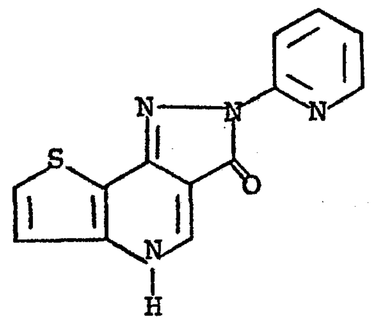

(E30)

The title compound was prepared in 60% yield from the chloro-ester D5 and 2-pyridylhydrazine using a method similar to that described in Examples 1 and 20.
For the cyclisation step two equivalents of potassium carbonate as base was used in sec butanol at reflux, for 20hr, under an atmosphere of nitrogen. m.p. > 340°.

Found: C, 53.90; H, 3.51; N, 19.78 $C_{13}H_8N_4OS.H_2O$
Requires: C, 54.50; H, 3.52 and N, 19.57%.

Found: $M^+$ 268.0409 Calc. for $C_{13}H_8N_4OS$ 268.0419.

Nmr ($d^6$DMSO) δ: 7.05-7.30 (1H, m), 7.40, 7.98 (2H, ABq, J=5.5), 7.80, 8.21 (2H, ABq, J=8.5), 8.45 (1H, m) and 8.70 (1H, s).

Example 31

2-(4-Chlorophenyl)-2,5-dihydro-3H-pyrazolo[3,4-d]thieno[3,4-b]pyridin-3-one (E31)

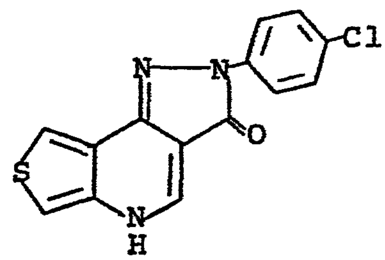

(E31)

Prepared by strict analogy to Example 30.

m.p. > 300$^{o}$ (dec.).

Found: $M^{+}$ 301.0075 Calc. for $C_{14}H_{8}N_{3}OSCl$ 301.0076

Nmr ($d_6$-DMSO) δ: 7.45 (2H,$d_t$, J=9,2), 7.66 (1H,d,J=3), 8.18 (2H,$d_t$, J=9,2), 8.26 (1H,d,J=3), 8.49 (1H,s).

Example 32

2-(4-Chlorophenyl)-2,5-dihydro-7-methyl-3H-pyrazolo[3,4-d]thieno[3,2-b]pyridin-3-one (E32)

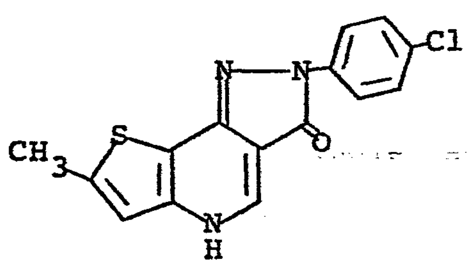

(E32)

Prepared by strict analogy to Example 30.

Example 33

2,5-Dihydro-2-(4-isopropylphenyl)-3H-pyrazolo[3,4-d]thieno[3,2-b]pyridin-3-one (E33)

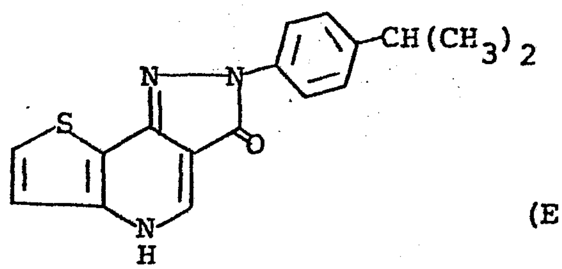

(E33)

Prepared by strict analogy to Examples 4 and 30.

Nmr ($d_6$DMSO) δ: 1.20 (6H,d,J=7), 2.87 (1H,m,J=7), 7.30 (2H,d,J=9), 7.40 (1H,d,J=5), 7.98 (1H,d,J=5), 8.12 (2H,d,J=9), 8.70 (1H,s).

Example 34

2-(4-Chlorophenyl)-2,5-dihydro-5-methyl-3H-pyrazolo[3,4-d] thieno[3,2-b]pyridin-3-one (E34)

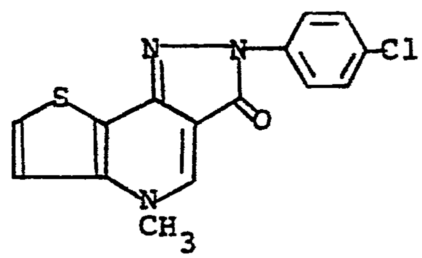

(E34)

To a stirred suspension of the chlorophenyl-pyrazolone prepared in Example 21 (1.00g, 3.3mM) in dry tetrahydrofuran (20ml) at room temperature, under nitrogen, was added 80% sodium hydride (0.12g, 4.0mM). Stirring was continued for 30 minutes. To the resulting clear solution was added methyl iodide (0.3ml, 4.8mM) over a period of 1 hour. Stirring at room temperature was continued for 18 hours. The resulting yellow solid was filtered off, washed with ether then recrystallised from tetrahydrofuran.

Found $M^+$ 315.0224

$C_{15}H_{10}N_3OSCl$ requires: 315.0233

Nmr (DMSO-$d_6$) δ: 4.07 (3H, s); 7.48 (2H, d, J=9Hz); 7.66 (1H, d, J=6Hz), 8.11 (1H, d, J=6Hz); 8.24 (2H, d, J=9Hz); 8.82 (1H, s).

Example 35

2-(4-Chlorophenyl)-1,2-dihydro-1-methyl-3H-pyrazolo[3,4-d]thieno[3,2-b]pyridin-3-one (E35)

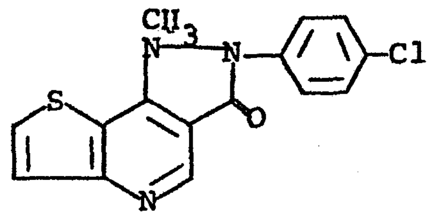

(E35)

A mixture of the chlorophenyl-pyrazolone prepared in Example 21, (0.15g, 0.5mm) and dimethylsulphate (4.5ml) was heated at 110°C for 18 hours. Excess dimethyl sulphate was evaporated in vacuo, and the residue partitioned between 10% aqueous sodium hydroxide (50ml) and dichloromethane (50ml). The organic phase was dried over anhydrous sodium sulphate and evaporated in vacuo to give a brown solid.

Found $M^+$ 315.0224

$C_{15}H_{10}N_3OSCl$ requires: 315.0233

NMR (DMSO-$d_6$) δ: 3.46 (3H, s); 7.64 (4H, s); 7.78 (1H,d, J=5Hz): 8.41 (1H,d,J=5Hz); 9.00 (1H,s).

0126970

Example 36

2-(4-Chlorophenyl)-2,5-dihydro-4-phenyl-3H-pyrazolo[3,4-d]thieno[3,2-b]pyridin-3-one (E36)

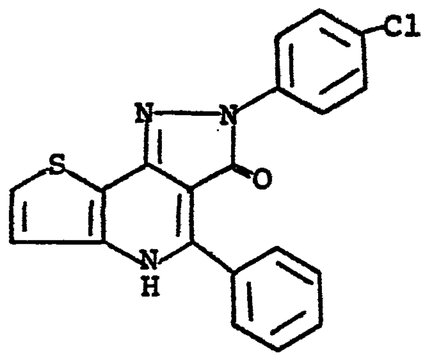

(E36)

The title compound was prepared from the chloro-ester D10 and 4-chlorophenylhydrazine in 50% yield using a method similar to that described in Examples 13 and 20.
m.p. 285-290°.

Found $M^+$ 377.0374

$C_{20}H_{12}N_3OSCl$ requires: 377.0388

Nmr (DMSO) δ: 7.40-8.40 complex multiplet

Examples 37 to 39

6-Chloro-2-phenyl-2,5-dihydro-3H-pyrazolo[3,4-d]thieno[3,2-b]pyridine-3-one (E37), 2,4-diphenyl-2,5-dihydro-3H-pyrazolo[3,4-d]thieno[3,2-b]pyridine-3-one (E38) and methyl-2-phenyl-2,5-dihydro-3H-pyrazolo[3,4-d]thieno[3,2-b]pyridine-3-one (E39) are prepared using a method similar to that described in Examples 20 and 9, 12 and 17 respectively.

Example 40

2-(4-Chlorophenyl)-2,5-dihydro-5-(3-dimethylaminopropyl)-3H-pyrazolo[3,4-d]thieno[3,2-b]pyridin-3-one (E40) was prepared analogously to Example 34.

Pharmacological Data

1. Anxiosoif Test

This behavioural paradigm used to predict anxiolytic activity is based on that described by Soubrie et al (1976). The method involves exposure of single naive 24hr water-deprived rats (♂ Hacking and Churchill CFHB) for 10 min to an illuminated novel environment comprising of a cylindrical perspex cage where water is available. Drinking behaviour is suppressed in these rats and anti-anxiety drugs (30 min pretreatment, i.p.) release the suppressed behaviour such that (i) the time spent drinking in a ten minute period and (ii) the total volume of water drunk during that period is increased. The results are expressed as a percentage change from control. 6 rats per treatment group are tested.

Soubrie et al., (1976)
Psychopharm., 50, 41-45

The results are shown in Table 1.

Toxicity

No toxic effects were observed in these tests.

Table 1

| Compound | Dose mg/kg i.p. | % change from control | |
|---|---|---|---|
| | | Time Spent drinking | Volume drunk |
| E1 | 20 | + 245 | + 133 |

## 2. Shock Induced Suppression of Drinking in the Rat

The shock induced suppression of drinking (SSD) test (adapted from Vogel *et al.*, 1971) is considered a reliable and specific method for showing up potential anxiolytics.

20h water-deprived rats (♂ Hacking and Churchill, CFHB), familiarised to the test apparatus the day before test, are allowed to drink for 30 sec and then receive 0.5m sec. footshock, maximum 0.5mA, for every 5 sec of drinking time accumulated in a 3 min session. Drugs were administered, intraperitoneally, 30 min before test.

The number of shocks taken during a given test period are recorded and results expressed as percentage change from control. Anxiolytic drugs such as benzodiazepines release the behaviour of rats suppressed in this way, such that the number of shocks taken increases in a dose dependent manner.

J.R. Vogel *et al.*, (1971) Psychopharmacologia (Berl.) 21, 1-7.

The results are shown in Table 2.

### Toxicity

No toxic effects were observed in these tests.

Table 2

| Compound | Dose mg/kg i.p. | % change from control no. of shocks taken |
|---|---|---|
| E1 | 20 | + 240 |
| E2 | 20 | + 92 |
| E3 | 10 | + 87 |
| E4 | 10 | + 175 |
| E20 | 20 | + 90 |
| E21 | 20 | + 202 |
| E22 | 10 | + 93 |
| E27 | 10 | + 160 |

3. Radioligand Binding Studies, in vitro

An interaction with benzodiazepine receptors in the central nervous system may be indicative of anxiolytic activity since inhibition of benzodiazepine binding correlates with the clinical efficacy of benzodiazepines. [$^3$H]-Flunitrazepam and [$^3$H]-βCCE selectively label benzodiazepine receptors and displacement of this specific binding in vitro by novel compounds in well-washed, frozen rat whole brain membranes is measured essentially as described by Martin and Candy (1978). At the fixed concentration of 0.5nM used, specific binding of both [$^3$H] ligands represents 80-90% of the total radioactivity bound. Non-specific binding is defined by 10μM clonazepam for each ligand. $IC_{50}$ values are calculated from log [dose] against % inhibition curves; Ki values are determined using the Cheng-Prusoff equation.

Martin, I.L. & Candy, J.M. (1978)
Neuropharm., 17, 993-998.

The results are shown in Table 3.

Table 3

| Compound | $[^3H]$-Flu Ki | $[^3H]$-βCCE Ki |
|---|---|---|
| E1 | 7µM | 5µM |
| E3 | 1µM | 1.5µM |
| E5 | 6.5µM | $>10^{-4}$M |
| E6 | 350nM | 360nM |
| E8 | 2.4µM | 3.3µM |
| E10 | 39µM | 23µM |
| E11 | 200nM | 290nM |
| E20 | 1µM | 2.2µM |
| E21 | 0.46nM | 0.38nM |
| E22 | 180nM | 170nM |
| E23 | 0.82nM | 0.31nM |
| E24 | 0.23nM | - |
| E25 | 99nM | 107nM |
| E28 | 1.8nM | - |
| E29 | 568nM | - |
| E30 | 0.7nM | - |
| E31 | 0.32nM | - |

CLAIMS

1. A compound of formula (I) or a pharmaceutically acceptable salt thereof:

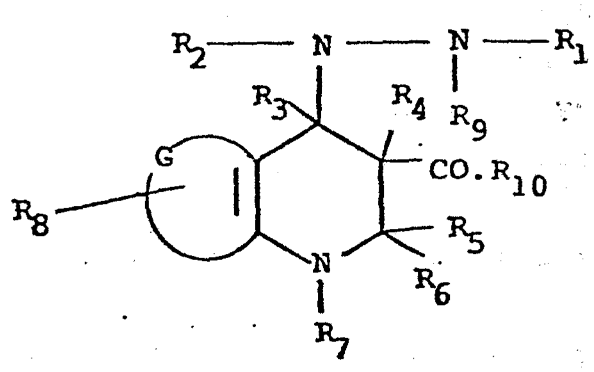

(I)

wherein:

G together with the two carbon atoms to which it is bonded is a thieno moiety;

$R_1$ is phenyl optionally substituted by one or more $C_{1-6}$ alkyl, $C_{1-6}$ alkoxy, $C_{1-6}$ alkylthio, hydroxy, $C_{2-7}$ alkanoyl, halo, trifluoromethyl, nitro, amino optionally substituted by one or two $C_{1-6}$ alkyl groups or by $C_{2-7}$ alkanoyl, cyano, carbamoyl or carboxy groups; or pyridyl optionally substituted by $C_{1-6}$ alkyl or halo;

$R_6$ is hydrogen, $C_{1-6}$ alkyl or phenyl optionally substituted as defined hereinbefore for $R_1$ when phenyl;

$R_8$ is hydrogen, one of the optional substituents recited hereinbefore for $R_1$ when phenyl or phenyl optionally substituted as defined hereinbefore for $R_1$ when phenyl; and either

$R_2$ is hydrogen, or $C_{1-6}$ alkyl optionally substituted by hydroxy, amino disubstituted by $C_{1-6}$ alkyl, or phenyl optionally substituted as defined hereinbefore for $R_1$ when phenyl;

$R_3$ and $R_4$ together represent a bond;

$R_5$ and $R_7$ together represent a bond; and

$R_9$ is hydrogen and $R_{10}$ is hydroxy, $C_{1-6}$ alkoxy or amino optionally substituted by one or two independently selected $C_{1-6}$ alkyl groups or by phenyl optionally substituted as defined herinbefore for $R_1$ when phenyl, or $R_9$ and $R_{10}$ together represent a bond;

or

$R_2$ and $R_3$ together represent a bond;

$R_4$ and $R_5$ together represent a bond; and

$R_7$ is hydrogen, $C_{1-6}$ alkyl optionally substituted by hydroxy, amino disubstituted by $C_{1-6}$ alkyl, or

phenyl optionally substituted as defined hereinbefore for $R_1$ when phenyl;

and

$R_9$ and $R_{10}$ together represent a bond.

2. A compound according to claim 1, wherein the thieno moiety formed by G and the two carbon atoms to which it is bonded is fused along its 2,3-face to the pyridine or dihydropyridine ring depicted in formula (I) in claim 1, in either the [2,3-b] or [3,2-b] orientation.

3. A compound according to claim 1 or 2 wherein the thieno moiety formed by G and the two carbon atoms to which it is bonded is fused along its 2,3-face to the pyridine or dihydropropyridine ring depicted in formula (I) in claim 1 in the [3,2-b] orientation.

4. A compound according to claim 1 wherein the thieno moiety formed by G and the two carbon atoms to which it is bonded is fused along its 3,4-face to the b-face of the pyridine or dihydropyridine ring depicted in formula (I) in claim 1.

5. A compound according to any preceding claim wherein $R_9$ and $R_{10}$ together represent a bond, $R_6$ is hydrogen or $C_{1-6}$ alkyl and $R_8$ is hydrogen or one of the

optional substituents recited in claim 1 for $R_1$ when phenyl.

6. A compound according to any one of claims 1 to 4 wherein $R_9$ is hydrogen and $R_{10}$ is hydroxy or $C_{1-6}$ alkoxy, $R_2$ is hydrogen and $R_6$ and $R_8$ are as defined in claim 5.

7. A compound according to any one of claims 1 to 4 and 6 which is
7-(2-phenylhydrazino)-thieno[3,2-b]pyridine-6-carboxylic acid, ethyl ester,
7-(4-chlorophenylhydrazino)-thieno[3,2-b]pyridine-6-carboxylic acid, ethyl ester,
7-(2-(4-nitrophenyl)hydrazino)-thieno[3,2-b]pyridine-6-carboxylic acid, ethyl ester,
7-(2-(4-isopropylphenyl)hydrazino)-thieno[3,2-b]-pyridine-6-carboxylic acid, ethyl ester,
7-(2-(4-methylphenyl)hydrazino)-thieno[3,2-b]pyridine-6-carboxylic acid, ethyl ester,
7-(2-(2-pyridyl)hydrazino)-thieno[3,2-b]pyridine-6-carboxylic acid, ethyl ester,
7-(2-(4-methoxyphenyl)hydrazino)-thieno[3,2-b]pyridine-6-carboxylic acid, ethyl ester,
5-methyl-7-(2-phenylhydrazino)-thieno[3,2-b]pyridine-6-carboxylic acid, ethyl ester, or
7-(2-(4-chlorophenyl)hydrazino)-5-methyl-thieno[3,2-b]-pyridine-6-carboxylic acid, ethyl ester, or a pharmaceutically acceptable salt thereof.

8. A compound according to any one of claims 1 to 5 which is
2-(4-chlorophenyl)-2,5-dihydro-4-methyl-3H-pyrazolo-[3,4-d]thieno[3,2-b]pyridin-3-one,
2-(4-chlorophenyl)-2,5-dihydro-3H-pyrazolo[3,4-d]-thieno[3,2-b]pyridin-3-one,
2-(3,5-dichlorophenyl)-2,5-dihydro-3H-pyrazolo[3,4-d]-thieno[3,2-b]pyridin-3-one,
2,5-dihydro-2-(4-methylphenyl)-3H-pyrazolo[3,4-d]-thieno-[3,2-b]pyridin-3-one,
2,5-dihydro-2-(4-methoxyphenyl)-3H-pyrazolo[3,4-d]-thieno[3,2-b]pyridin-3-one,
2,5-dihydro-4-methyl-2-phenyl-3H-pyrazolo[3,4-d]thieno-[3,2-b]pyridin-3-one,
2-(4-chlorophenyl)-2,5-dihydro-4,8-dimethyl-3H-pyrazolo-[3,4-d]thieno[2,3-b]pyridin-3-one,
2-(4-chlorophenyl)-2,5-dihydro-7-ethyl-3H-pyrazolo-[3,4-d]thieno[2,3-b]pyridin-3-one,
2-(4-chlorophenyl)-2,5-dihydro-7-ethyl-4-methyl-3H-pyrazolo[3,4-d]thieno[2,3-b]pyridin-3-one,
2,5-dihydro-2-(2-pyridyl)-3H-pyrazolo[3,4-d]thieno-[3,2-b]pyridin-3-one, or
2-(4-chlorophenyl)-2,5-dihydro-3H-pyrazolo[3,4-d]-thieno-[3,4-b]pyridin-3-one, or a pharmaceutically acceptable salt thereof.

9 A pharmaceutical composition, which comprises a compound according to any one of claims 1 to 8 of formula (I) or a pharmaceutically acceptable salt thereof, and a pharmaceutically acceptable carrier.

10 A process for the preparation of a compound according to any one of claims 1 to 8 of formula (I) or a pharmaceutically acceptable salt thereof which process comprises the reaction of a compound of formula (XVI):

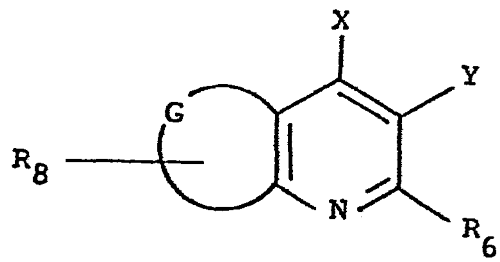

(XVI)

or a salt thereof,

wherein

i) when $R_9$ and $R_{10}$ in the desired compound of formula (I) are hydrogen, and $R_{10}^1$ respectively, where $R_{10}^1$ is hydroxy, $C_{1-6}$ alkoxy or amino optionally substituted by one or two independently selected $C_{1-6}$ alkyl groups or by phenyl optionally substituted as defined in claim 1 for $R_1$ when phenyl;

X is halo;

Y is $COR_{10}^1$ as hereinbefore defined or nitrile; and the remaining variables are as defined in claim 1;

with a compound of formula $R_2HN-NH-R_1$ where $R_1$ and $R_2$ are as defined in claim 1;

and thereafter, in the resultant compound, when Y is nitrile, converting Y to $COR_{10}{}^1$ as hereinbefore defined; and optionally converting $R_{10}{}^1$ to other $R_{10}{}^1$; when $R_9$ and $R_{10}$ in the desired compound of

ii)

formula

(I) together represent a bond;

a) X is $NR_2-NH-R_1$ where $R_1$ and $R_2$ are as hereinbefore defined and Y is $COR_{11}$ where $R_{11}$ is halo or Y is $COR_{10}{}^2$ where $R_{10}{}^2$ is hydroxy or $C_{1-6}$ alkoxy and the compound of formula (XVI) or the salt thereof is optionally prepared by process variant i) hereinbefore optionally followed by salification;

b) X is halo, and Y is $CON(NR_2R_{15})R_1$ where $R_1$ and $R_2$ are as hereinbefore defined; and $R_{15}$ is hydrogen or a labile deactivating N-protecting group;

c) X is $C_{1-6}$ alkoxyamino or azido and Y is $CONHR_1$ where $R_1$ is as hereinbefore defined;

to cyclise;

and thereafter, in the resultant compound of formula (I) wherein $R_9$ and $R_{10}$ together represent a bond; optionally converting $R_2$ or $R_7$ hydrogen to other $R_2$ or $R_7$;

and, in the resultant compound of formula (I), optionally converting $R_8$ to other $R_8$; and optionally forming a pharmaceutically acceptable salt.

11 A compound of formula (XVI) as in claim 10, wherein the variables are as defined in claim 10.

12 A compound according to any one of claims 1 to 8 of formula (I) or a pharmaceutically acceptable salt thereof, for use in the treatment of CNS disorders, in particular anxiety or depression.